# EUROPEAN PATENT SPECIFICATION

(11) **EP 4 368 469 B1**
(45) Date of publication and mention of the grant of the patent: **15.04.2026**
(21) Application number: 24167438.1
(22) Date of filing: 10.08.2018
(51) Int. Cl.: G08B 21/06, A61B 5/18, A61B 3/113, A61B 5/11, A61B 5/16, B60W 40/08, B60W 50/08

(54) **VISUAL LINE MOVEMENT-RELATED VALUE ACQUISITION DEVICE, AND TRANSPORT APPARATUS EQUIPPED WITH SAME**
VORRICHTUNG ZUR ERFASSUNG VON WERTEN IM ZUSAMMENHANG MIT DER BEWEGUNG EINER VISUELLEN LINIE UND TRANSPORTVORRICHTUNG DAMIT
DISPOSITIF D'ACQUISITION DE VALEUR RELATIVE AU MOUVEMENT D'UNE LIGNE VISUELLE, ET APPAREIL DE TRANSPORT ÉQUIPÉ DE CELUI-CI

(30) Priority: 10.08.2017 JP 2017155947
(43) Date of publication of application: 15.05.2024
(62) Divisional of application: 18844612.4
(73) Proprietor: Yamaha Hatsudoki Kabushiki Kaisha, Iwata-shi, Shizuoka 438-8501 (JP)
(72) Inventor: MORISHIMA, Keisuke, Iwata-shi, Shizuoka, 438-8501 (JP); YAMANAKA, Kimihiro, Kobe-shi, Hyogo, 658-8501 (JP); CHIHARA, Takanori, Kanazawa-shi, Ishikawa, 920-1192 (JP)
(74) Representative: Yoshida, Senko

(56) References cited:
- JP-A- 2017 094 121
- US-B2- 8 849 474

## Description

### TECHNICAL FIELD

The present teaching relates to a visual-line-movement-related-value-acquisition device that acquires a visual line movement-related value related to a visual line movement of a subject, and a transport apparatus equipped with the visual-line-movement-related-value-acquisition device.

### BACKGROUND ART

As a device that estimates a state of a person, based on eye motion and head motion of the person, for example, a person's-state-estimation device disclosed in Patent Document 1 has been known. The person's-state-estimation device compares a timing of eye motion of a person and a timing of head motion of the person with each other to thus determine an order of the timings and estimates an internal state of the person, based on a determination result.

Specifically, the person's state-estimation-device disclosed in Patent Document 1 estimates an internal state of a person in accordance with the steps illustrated in FIG. 17. The steps will be described below by citing paragraphs [0062] to [0082] of Patent Document 1.

First, in Steps S1 and S2, eye motion and head motion are measured. Specifically, an eye camera measures the eye motion. The measurement result is time series data of an eye angle (information related to an eye rotation angle at each time).

A gyro sensor measures the head motion. The measurement result is time series data related to an angular velocity and an angle of the head (information related to an angular velocity and an angle of the head at each time).

The eye camera and the gyro sensor simultaneously perform measurements. Thus, respective times of the time series data can be easily synchronized.

Next, in Step S3, a timing of the eye motion is specified. Specifically, an eye-motion-timing-specifying section specifies the timing of the eye motion, based on the measurement result of the eye camera. Eye motion that satisfies a predetermined condition is selected and a timing of the selected eye motion is specified. Processing of extracting eye motion and processing of specifying a timing of the eye motion will be separately described below.

In the processing of extracting eye motion, saccade (saccadic eye motion) is selected. The saccade is also called "rapid eye motion" and is eye motion that quickly moves eyes.

The saccade is defined as eye motion in which an eye movement amount is a predetermined value De or more, and an eye angular velocity is a predetermined angular velocity Re or more, and eye motion that satisfies all of these conditions is extracted. The eye movement amount is an eye rotation angle in one eye motion and is also called amplitude. The eye movement amount is a movement amount in a period in which the eye angular velocity exceeds the predetermined angular velocity Re. The predetermined value De is, for example, 3.5 degrees. The angular velocity Re is, for example, 30 degree/sec.

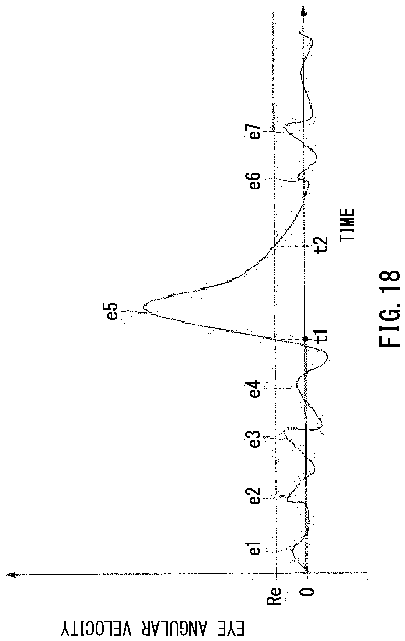

The process of extracting eye motion will be described with reference to FIG. 18. In FIG. 18, the abscissa indicates a time and the ordinate indicates eye angular velocity. In FIG. 18, for convenience of description, the eye angular velocity in arbitrary one direction is indicated. Note that the eye angular velocity can be obtained by differentiating a measurement result of the eye camera by time.

FIG. 18 illustrates a plurality of eye motions e1 to e7. Among the eye motions e1 to e7, only eye motion e5 has an eye angular velocity equal to or more than the predetermined angular velocity Re. Therefore, the other eye motions e1 to e4, e6, and e7 are not extracted (excluded). For the eye motion e5, an eye movement amount is obtained. In this case, the eye movement amount is obtained by integrating the eye angular velocity by a period from a time t1 to a time t2 in which the eye angular velocity exceeds the predetermined angular velocity Re. Then, when the obtained eye movement amount is the predetermined value De or more, the eye motion e5 is extracted and when not, the eye motion e5 is not extracted.

Next, the processing of specifying a timing of eye motion will be described. The timing of the eye motion is a start time of the eye motion. Also, the start time is the beginning of a period in which the angular velocity exceeds the predetermined angular velocity Re. For the eye motion e5 illustrated in FIG. 18, the timing is specified to be the time t1.

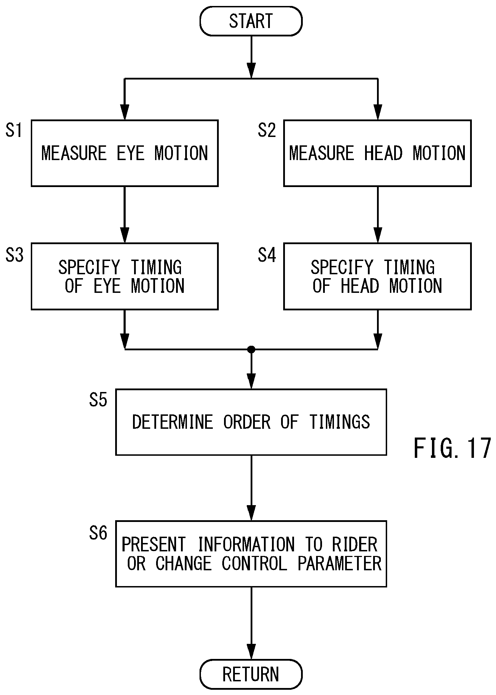

In Step S4 illustrated in FIG. 17, a timing of the head motion is specified. Specifically, a head-motion-timing-specifying section specifies the timing of the head motion, based on the measurement result of the gyro sensor.

The head-motion-timing-specifying section selects head motion in which a maximum value of a head angular acceleration is a predetermined angular acceleration Ah or more, and a timing of the selected head motion is calculated. In this case, the timing of the head motion is a start time of the head motion, and the start time is the beginning of a period in which the head angular acceleration is a predetermined threshold Th or more. The threshold Th is, for example, 0 degree/sec².

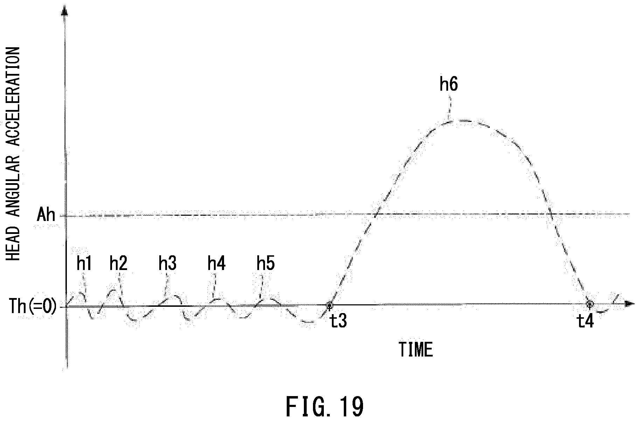

The processing of specifying a timing of head motion will be described with reference to FIG. 19. In FIG. 19, the abscissa indicates a time and the ordinate indicates a head angular acceleration. In FIG. 19, for convenience of description, the head angular acceleration in arbitrary one direction is indicated.

FIG. 19 illustrates a plurality of head motions h1 to h6. Among the head motions h1 to h6, only the head motion h6 includes a zone in which the head angular acceleration is equal to or more than the predetermined angular acceleration Ah. In the head motion h6, a period in which the angular acceleration exceeds the threshold Th is from a time t3 to a time t4. Therefore, the time t3 is specified to be a timing of the head motion h6. Note that the other head motions h1 to h5 are not extracted and the timings of the head motions h1 to h5 are not specified.

In Step S5 illustrated in FIG. 17, an order of timings is determined. Specifically, a timing comparison and estimation section compares respective timings of eye motion and head motion with each other and determines the order of the timings.

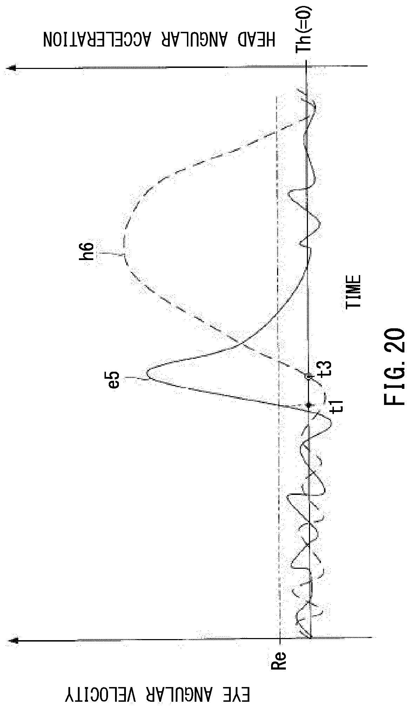

See FIG. 20. FIG. 20 is a graph illustrating a timing of eye motion and a timing of head motion. FIG. 20 is a graph obtained by overlaying FIG. 18 and FIG. 19. When the timings have a relationship illustrated in FIG. 20, it is determined that the timing of the eye motion (a start time t1) precedes the timing of the head motion (a start time t3). Thus, it is understood that a viewing action is an eye preceding type.

As described above, the order information is an evaluation index indicating an internal state of a rider. For example, the order information indicating the eye preceding type means that an attention level of the rider is relatively high (or that it can be estimated that the attention level of the rider is relatively high). On the other hand, the order information indicating a head preceding type means that the attention level of the rider is relatively low. A state in which "the attention level is relatively high" is not a state in which an excessive attention is paid to a specific event but a state in which attentions can be paid to various events. A state in which "the attention level is relatively low" is a state in which an excessive attention is paid to a specific event (including a plurality of events) and attentions to other events are lost.

The timing-comparison-and-estimation section stores the order information in a storage and outputs the order information to an output selection section.

In Step S6 illustrated in FIG. 17, information is presented to the rider or a control parameter is changed. Specifically, the output selection section makes a selection related to presentation of information and change of the control parameter based on the order information, and appropriately outputs an order to a display section, a resistance adjustment section, a vibration generation section, an audio output section, and an ECU. As a result of the selection, there is not only a case in which the order is output to all or some of the display section, the resistance adjustment section, the vibration generation section, the audio output section, and the ECU, but also a case in which the order is not output to any one of those sections.

When the output selection section outputs the order to at least one of the display sections, the resistance adjustment section, the vibration generation section, and the audio output section, an estimation result for an internal state of the rider or information related to advice to the rider or the like is presented. For example, the display section may be configured to display a numerical value, a graph, or the like, indicating the estimation result for the internal state of the rider. The resistance adjustment section may be configured to change a resistance to a rotation of an accelerator grip. The vibration generation section may be configured to vibrate a seat. The audio output section may be configured to output a voice saying, "Shall we take a break now?" or the like.

When the output selection section outputs the order to the ECU, the ECU changes a control parameter for a straddled vehicle. Due to this change of the control parameter, controllability (easiness of control) of the straddled vehicle is forcibly adjusted. For example, the controllability of the straddled vehicle may be simplified to reduce a burden of the rider. When a beginner mode and a proficient mode are set in advance, the modes may be switched from one to another.

Then, returning to Step S1, a series of processing described above is repeated.

As described above, based on a finding that the internal state of the rider can be estimated by determining whether a viewing action is the eye preceding type or the head preceding type, only eye motion and head motion that can be measured in a relatively easy manner is used as biological information. Thus, an eye camera or a simple sensor such as a gyro sensor, can measure eye motion and head motion. Furthermore, the timing comparison and estimation section can obtain the order information on which the internal state of the rider is reflected by relatively simple processing, such as determination of the order of respective timings of eye motion and head motion. As described above, the internal state of the rider can be easily estimated based on the biological information that can be easily measured.

In Patent Document 1, it is disclosed that a time difference between respective timings of eye motion and head motion is an evaluation index more specifically indicating the internal state of the rider, as compared to the order information. Further, Patent Document 2 discloses a human state estimation apparatus comprising: an eye camera for measuring an ocular movement; a gyro sensor for measuring a head movement; an ocular movement timing specification part for specifying timing of the ocular movement on the basis of a measurement result by the eye camera; a head movement timing specification part for specifying timing of the head movement on the basis of a measurement result by the gyro sensor; and a timing comparison/estimation part for determining the order of these timing by comparing the timing of the ocular movement with the timing of the head movement, and estimating a human internal state on the basis of this determination result. In addition, Patent Document 3 discloses a rider characteristic determining apparatus for determining characteristics of a rider controlling a saddle riding type vehicle, the rider characteristic determining apparatus are configured such that the rider's characteristics are determined from a turning movement of the saddle riding type vehicle which reflects results of the rider controlling the saddle riding type vehicle. A turning performance score of the vehicle is calculated based on at least one of vehicle state amounts of a roll direction, a pitch direction and a caster angle which influence the steering angle of the saddle riding type vehicle.

On the other hand, it has been known that eye motion and head motion are related to an effective visual field that changes in accordance with an internal state of a person. As a document that describes this relationship, for example, Non-patent Document 1 or the like has been known. In Non-patent Document 1, it is suggested that an effective visual field can be detected from a time difference between eye motion and head motion.

### CITATION LIST

### PATENT DOCUMENT

Patent Document 1: Japanese Patent Application Publication No. 2014-113227
Patent Document 2: Japanese Patent Application Publication No. 2017-94121
Patent Document 3: United States Patent No. 8 849 474

### NON-PATENT DOCUMENT

"Gankyu·Toubu Kyocho Undou To Yuko Shiya No Kankei (Relationship Between Eye and Head Coordinated motion and Effective Visual Field)," Keisuke Morishima and three others, 2016, Vol. 67, No. 3, p.252-260, Journal of Japan Industrial Management Association, Japan Industrial Management Association.

### SUMMARY OF INVENTION

### PROBLEMS TO BE SOLVED BY INVENTION

An effective visual field is related to an internal state of a person and therefore, in order to understand the internal state of the person, it is effective to detect the effective visual field. However, the internal state of the person largely differs among individuals and furthermore, the internal state of the person largely changes in accordance with the situation in which the person is put. Therefore, it is difficult to detect the effective visual field that changes in accordance with the internal state.

It is therefore an object of the present teaching to achieve a structure in which an effective visual field of a person can be detected.

### SOLUTION TO PROBLEM

The present inventors found through their vigorous examinations of the above-described method of Patent Document 1 for estimating an internal state of a person using eye motion and head motion that an effective visual field can be relatively evaluated using the eye motion and the head motion as disclosed in Non-patent Document 1.

However, the internal state of the person largely differs among individuals and furthermore, the internal state of the person largely changes in accordance with the situation in which the person is put. Therefore, it is difficult to detect the effective visual field that changes in accordance with the internal state of the person.

The present inventors noticed through further examinations on influences of individual differences on changes of the internal state of the person that data is acquired from a subject and thereby, data in consideration of individual characteristics of the subject can be acquired.

Specifically, the present inventors arrived at the structures below. The present invention is defined by the appended claims.

A visual-line-movement-related-value-acquisition device according to an embodiment of the present teaching acquires a subject's visual line movement-related value related to a visual line movement of a subject, acquires a coordinated motion-related value related to a coordinated motion of eye motion and head motion of the subject for the acquired subject's visual line movement-related value, and outputs the subject's visual line movement-related value related to the visual line movement of the subject when the coordinated motion-related value related to the coordinated motion of the eye motion and the head motion of the subject is a predetermined value from a relationship between the acquired subject's visual line movement-related value related to the visual line movement of the subject and the acquired coordinated motion-related value related to the coordinated motion of the eye motion and the head motion of the subject.

Thus, the subject's visual line movement-related value and the coordinated motion-related value of the subject can be acquired. Moreover, in the relationship between the subject's visual line movement-related value and the coordinated motion-related value, the subject's visual line movement-related value when the coordinated motion-related value is the predetermined value is output, and thereby, an index related to the visual line movement of the subject can be obtained. It is then possible to obtain an index related to an effective visual field of the subject from the index related to the visual line movement of the subject.

According to another aspect, the visual-line-movement-related-value-acquisition device of the present teaching preferably includes a structure below. The visual-line-movement-related-value-acquisition device continuously acquires the same subject's continuous data including a plurality of data related to a same subject, acquires a value related to the visual line movement of the subject as the subject's visual line movement-related value from the same subject's continuous data, and acquires, as the coordinated motion-related value, a value related to the coordinated motion of the eye motion and the head motion of the subject for the acquired subject's visual line movement-related value from the same subject's continuous data.

Thus, the subject's visual line movement-related value and the coordinated motion-related value less influenced by individual differences of the subject can be acquired. Moreover, in the relationship between the subject's visual line movement-related value and the coordinated motion-related value, the subject's visual line movement-related value when the coordinated motion-related value is the predetermined value is output and thereby, an index related to the visual line movement of the subject can be acquired without being influenced by individual differences. It is then possible to obtain an index related to the effective visual field of the subject from the index related to the visual line movement of the subject.

According to another aspect, the visual-line-movement-related-value-acquisition device of the present teaching preferably includes a structure below. The visual-line-movement-related-value-acquisition device arranges the acquired coordinated motion-related values related to the coordinated motion of the eye motion and the head motion of the subject for the subject's visual line movement-related value related to the visual line movement of the subject in an order of magnitude of the acquired subject's visual line movement-related value related to the visual line movement of the subject, extracts a predetermined number of consecutive values from the coordinated motion-related values related to the coordinated motion of the eye motion and the head motion of the subject, the coordinated motion-related values being arranged in the order, and calculates a representative value of the coordinated motion-related values and also calculates, from the same subject's visual line movement-related values related to the visual line movement of the subject, the same subject's visual line movement-related values corresponding to the extracted coordinated motion-related values related to the coordinated motion of the eye motion and the head motion of the subject, a representative value of the same subject's visual line movement-related values, and acquires the relationship between the subject's visual line movement-related value related to the visual line movement of the subject and the coordinated motion-related value related to the coordinated motion of the eye motion and the head motion of the subject from the calculated representative value of the coordinated motion-related values related to the coordinated motion of the eye motion and the head motion of the subject and the calculated representative value of the subject's visual line movement-related values related to the visual line movement of the subject.

Thus, the relationship between the subject's visual line movement-related value and the coordinated motion-related value can be easily obtained. Therefore, an index related to the effective visual field of the subject can be easily obtained without being influenced by individual differences.

According to another aspect, the visual-line-movement-related-value-acquisition device of the present teaching preferably includes a structure below. The subject's visual line movement-related value related to the visual line movement of the subject is a visual line movement amount of the subject.

Thus, as a parameter related to the effective visual field of the subject, a movement amount of a visual line (a visual line movement amount) when the coordinated motion-related value related to the coordinated motion of the eye motion and the head motion of the subject is a predetermined value can be obtained.

According to another aspect, the visual-line-movement-related-value-acquisition device of the present teaching preferably includes a structure below. The coordinated motion-related value related to the coordinated motion of the eye motion and the head motion of the subject is a head motion preceding ratio that is a ratio at which the head motion precedes the eye motion in the coordinated motion or a motion time difference that is a time difference between a start timing of the head motion and a start timing of the eye motion in the coordinated motion.

In the coordinated motion of the eye motion and the head motion of the subject, the head motion preceding ratio, at which the head motion precedes the eye motion or the motion time difference that is a time difference between a start timing of the head motion and a start timing of the eye motion, changes in accordance with an internal state of a person. Therefore, the effective visual field related to the internal state of the person can be detected by calculating the subject's visual line movement-related value related to the visual line movement of the subject when the head motion preceding ratio or the motion time difference is a predetermined value using the head motion preceding ratio or the motion time difference as a parameter.

According to another aspect, the visual-line-movement-related-value-acquisition device of the present teaching preferably includes a structure below. As the predetermined value of the coordinated motion-related value related to the coordinated motion of the eye motion and the head motion of the subject, the head motion preceding ratio is 50%

Thus, when using the head motion preceding ratio as a parameter, the effective visual field related to the internal state of the person can be detected with high accuracy.

A visual-line-movement-related-value-acquisition device according to an embodiment of the present teaching includes a subject's-visual-line-movement-related-value-acquisition section configured to acquire a subject's visual line movement-related value related to a visual line movement of a subject, a coordinated motion-related value-acquisition section configured to acquire a coordinated motion-related value related to a coordinated motion of eye motion and head motion of the subject for the subject's visual line movement-related value acquired by the subject's-visual-line-movement-related-value-acquisition section, and a subject's-visual-line-movement-related-value-output section configured to output the subject's visual line movement-related value related to the visual line movement of the subject when the coordinated motion-related value related to the coordinated motion of the eye motion and the head motion of the subject is a predetermined value from a relationship between the subject's visual line movement-related value related to the visual line movement of the subject, the subject's visual line movement-related value having been acquired by the subject's-visual-line-movement-related-value-acquisition section, and the coordinated motion-related value related to the coordinated motion of the eye motion and the head motion of the subject, the coordinated motion-related value having been acquired by the coordinated motion-related value-acquisition section.

As for a transport apparatus according to an embodiment of the present teaching, the subject is a rider. When the subject is the rider of the transport apparatus, an index related to the visual line movement of the rider can be obtained without being influenced by individual differences using the above-described visual-line-movement-related-value-acquisition device. Therefore, an index related to an effective visual field of the rider can be obtained from the index related to the visual line movement of the rider.

According to another aspect, the transport apparatus according to the present teaching preferably includes a structure below. The transport apparatus includes a transport-apparatus-motion-measurement section configured to measure motion of the transport apparatus, and a head-motion-measurement section configured to measure head motion of the subject. The visual-line-movement-related-value-acquisition device excludes the motion of the transport apparatus measured by the transport-apparatus-motion-measurement section, from the head motion of the subject measured by the head-motion-measurement section, and calculates the subject's visual line movement-related value related to the visual line movement of the subject and the coordinated motion-related value related to the coordinated motion of the eye motion and the head motion of the subject.

Thus, an influence of the motion of the transport apparatus on the measured value can be excluded from the measured value of the head motion of the subject that is the rider of the transport apparatus. Therefore, the effective visual field of the subject can be detected with high accuracy.

According to another aspect, the transport apparatus according to the present teaching preferably includes a structure below. The transport apparatus includes an information output section configured to output information corresponding to the subject's visual line movement-related value related to the visual movement of the subject, the subject's visual line movement-related value having been output from the visual-line-movement-related-value-acquisition device.

Thus, it is possible to inform the subject or the like of information, based on the subject's visual line movement-related value obtained by the visual-line-movement-related-value-acquisition device as a parameter related to the effective visual field of the subject. Therefore, it is possible to inform the subject or the like of the internal state or the like.

According to another aspect, the transport apparatus according to the present teaching preferably includes a structure below. The transport apparatus includes a control device configured to control an operation of the transport apparatus, based on the subject's visual line movement-related value related to the visual line movement of the subject, the subject's visual line movement-related value having been output from the visual-line-movement-related-value-acquisition device.

Thus, an operation of the transport apparatus, a rider of which is the subject, can be controlled based on the subject's visual line movement-related value obtained by the visual-line-movement-related-value-acquisition device as a parameter related to the effective visual field of the subject. Therefore, the operation of the transport apparatus can be controlled in accordance with the internal state of the subject or the like.

A visual line movement-related value acquisition method according to an embodiment of the present teaching includes acquiring a subject's visual line movement-related value related to a visual line movement of a subject, acquiring a coordinated motion-related value related to a coordinated motion of eye motion and head motion of the subject for the acquired subject's visual line movement-related value, and outputting the subject's visual line movement-related value related to the visual line movement of the subject, when the coordinated motion-related value related to the coordinated motion of the eye motion and the head motion of the subject is a predetermined value from a relationship between the acquired subject's visual line movement-related value related to the visual line movement of the subject and the acquired coordinated motion-related value related to the coordinated motion of the eye motion and the head motion of the subject.

According to another aspect, the visual line movement-related value acquisition method according to the present teaching preferably includes a structure below. The visual line movement-related value acquisition method includes continuously acquiring same subject' continuous data including a plurality of data related to a same subject acquiring, as the subject's visual line movement-related value, a value related to the visual line movement of the subject from the same subject's continuous data, and acquiring, as the coordinated motion-related value, a value related to the coordinated motion of the eye motion and the head motion of the subject for the acquired subject's visual line movement-related value.

According to another aspect, the visual line movement-related value acquisition method according to the present teaching preferably includes a structure below. The visual line movement-related value acquisition method includes arranging the acquired coordinated motion-related values related to the coordinated motion of the eye motion and the head motion of the subject for the subject's visual line movement-related value related to the visual movement of the subject in an order of magnitude of the acquired subject's visual line movement-related value related to the visual movement of the subject, extracting a predetermined number of consecutive values from the coordinated motion-related values related to the coordinated motion of the eye motion and the head motion of the subject, the coordinated motion-related values being arranged in the order, and calculating a representative value of the coordinated motion-related values and also calculating, from the subject's visual line, movement-related values related to the visual line movement of the subject, the subject's visual line movement-related values corresponding to the extracted coordinated motion-related values related to the coordinated motion of the eye motion and the head motion of the subject, a representative value of the subject's visual line movement-related values, and acquiring the relationship between the subject's visual line movement-related value related to the visual movement of the subject and the coordinated motion-related value related to the coordinated motion of the eye motion and the head motion of the subject from the calculated representative value of the coordinated motion-related values related to the coordinated motion of the eye motion and the head motion of the subject and the calculated representative value of the subject's visual line movement-related values related to the visual movement of the subject.

According to another aspect, the visual line movement-related value acquisition method according to the present teaching preferably includes a structure below. The subject's visual line movement-related value related to the visual line movement of the subject is a visual line movement amount of the subject.

According to another aspect, the visual line movement-related value acquisition method according to the present teaching preferably includes a structure below. The coordinated motion-related value related to the coordinated motion of the eye motion and the head motion of the subject is a head motion preceding ratio that is a ratio at which a start of the head motion precedes a start of the eye motion in the coordinated motion or a motion time difference that is a time difference between a start timing of the head motion and a start timing of the eye motion in the coordinated motion.

According to another aspect, the visual line movement-related value acquisition method according to the present teaching preferably includes a structure below. As the predetermined value of the coordinated motion-related value related to the coordinated motion of the eye motion and the head motion of the subject, the head motion preceding ratio is 50%.

The terminology used herein is used for the purpose of defining some particular embodiments only and is not intended to limit the present teaching.

As used herein, the term "and/or" includes any and all combinations of one or more of the associated listed items.

It will be further understood that the terms "including" "comprising," or "having" and the variations thereof when used in this specification, specify the presence of stated features, steps, elements, components, and/or their equivalents, but can include steps, operations, elements, components, and/or one or more of the groups.

As used herein, the terms "attached," "connected," " combined," and/or the equivalents thereof are used in a broad sense to embrace both "direct and indirect" attachment, connection, and combination. Furthermore, the terms "connected" and "combined" are not limited to physical or mechanical connection or combination and can include direct or indirect connection or combination.

Unless otherwise defined, all the terms (including technical and scientific terms) used herein have the same meaning as commonly understood by one having ordinary skill in the art to which this present teaching belongs.

It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In describing the present teaching, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the present teaching.

Embodiments of a visual-line-movement-related-value-acquisition device according to the present teaching will be described.

In the following description, numerous specific examples are set forth in order to provide a thorough understanding of the present teaching. It will be evident, however, to one skilled in the art that the present teaching may be practiced without these specific examples.

Therefore, the following disclosure is to be considered as an exemplification of the present teaching, and is not intended to limit the present teaching to the specific embodiments illustrated by the figures or description below.

### [Effective Visual Field]

As used herein, the term "effective visual field" is a range in which a subject can collect visual information effectively used in an action of capturing a visual object in a field of vision.

### [Representative Value]

As used herein, the term "representative value" means a value, such as an average or a median of a plurality of values, which can be used for representing the plurality of values.

### [Visual Line Movement Amount]

As used herein, the term "visual line movement amount" is a value obtained by summing an eye movement amount that is a movement amount of eyes and a head movement amount that is a movement amount of a head.

### [Eye Motion]

As used herein, the term "eye motion" means motion of eyes relative to a head. That is, the eye motion means movement of eyes relative to a head. The eye motion includes saccade (saccadic eye motion) in which, for example, an absolute value of an eye rotation angular velocity is a threshold α (for example, 30 deg/sec) or more, and an absolute value of a motion amount obtained by integrating the rotation angular velocity is a threshold β (for example, 3.5 deg) or more.

### [Head Motion]

As used herein, the term "head motion" means motion of a head when the head moves or rotates in absolute coordinates in an up-down direction and a left-right direction. That is, the head motion includes motion of the head when the head moves or rotates relative to a body, motion of the head when the head and the body move or rotate together, and motion of the head when the head and the body separately move or rotate.

### [Start of Eye Motion]

As used herein, the term "start of eye motion" means a time point at which a parameter related to eye motion exceeds a predetermined threshold.

### [Start of Head Motion]

As used herein, the term "start of head motion" means a time point at which a parameter related to head motion exceeds a predetermined threshold.

### [Coordinated Motion of Eye Motion and Head Motion]

As used herein, the term "coordinated motion of eye motion and head motion" is motion including at least one of eye motion and head motion in a viewing action when a subject views a visual object.

### [To Continuously Acquire]

As used herein, the term "to continuously acquire" means to continuously acquire data in a period in which a relationship between a subject's visual line movement-related value related to the visual line movement of the subject and a coordinated motion-related value related to a coordinate motion of eye motion and head motion of the subject can be obtained.

### [Transport Apparatus]

As used herein, the term "transport apparatus" is not limited to a motorcycle and means a vehicle, such as a three-wheeled vehicle, a four-wheeled vehicle, or the like, a ship, an aircraft, or the like, which is driven and operated by a rider, a driver, or an occupant, and thereby can transport a transport object, such as a person, a load, or the like.

### ADVANTAGEOUS EFFECTS OF INVENTION

According to an embodiment of the present teaching, a structure in which an effective visual field of a person can be detected can be achieved.

### BRIEF DESCRIPTION OF DRAWINGS

[FIG. 1] FIG. 1 is a view illustrating an overall structure of a vehicle including a visual-line-movement-related-value-acquisition device according to a first embodiment of the present teaching.
[FIG. 2] FIG. 2 is a view schematically illustrating an effective visual field of a rider who drives a vehicle.
[FIG. 3] FIG. 3 is a view schematically illustrating an example of a viewing action.
[FIG. 4] FIG. 4 is a functional block diagram illustrating a structure of the visual-line-movement-related-value-acquisition device.
[FIG. 5] FIG. 5 is a graph illustrating an example of time change of eye rotation angular velocity.
[FIG. 6] FIG. 6 is a graph illustrating an example of time change of head rotation angular velocity.
[FIG. 7] FIG. 7 is a view schematically illustrating data processing of preceding motion information and visual line movement amount.
[FIG. 8] FIG. 8 is a graph illustrating an example of a relationship between head motion preceding ratio and visual line movement amount.
[FIG. 9] FIG. 9 is a graph illustrating a relationship between head motion preceding ratio and visual line movement amount in which an approximate curve is indicated.
[FIG. 10] FIG. 10 is a flowchart illustrating an operation of the visual-line-movement-related-value-acquisition device.
[FIG. 11] FIG. 11 is a functional block diagram illustrating a structure of a visual-line-movement-related-value-acquisition device according to a second embodiment.
[FIG. 12] FIG. 12 is a flowchart illustrating an operation of the visual-line-movement-related-value-acquisition device according to the second embodiment.
[FIG. 13] FIG. 13 illustrates a functional block diagram of a structure of the visual-line-movement-related-value-acquisition device according to the first embodiment, and a graph illustrating an example of a relationship between head motion preceding rate and visual line movement amount.
[FIG. 14] FIG. 14 is a functional block diagram illustrating a structure of a visual-line-movement-related-value-acquisition device according to a third embodiment.
[FIG. 15] FIG. 15 is a flowchart illustrating an operation of the visual-line-movement-related-value-acquisition device according to the third embodiment.
[FIG. 16] FIG. 16 is a functional block diagram illustrating a structure of a visual-line-movement-related-value-acquisition device according to a fourth embodiment.
[FIG. 17] FIG. 17 is a flowchart illustrating processing steps of a state estimation device in a known example.
[FIG. 18] FIG. 18 is a graph illustrating a relationship between an angular velocity of eye motion and a time in the known example.
[FIG. 19] FIG. 19 is a graph illustrating a relationship between an angular acceleration of head motion and a time in the known example.
[FIG. 20] FIG. 20 is a graph illustrating a timing of eye motion and a timing of head motion in the known example.

### DESCRIPTION OF EMBODIMENTS

Each embodiment will be described below with reference to the drawings. Note that dimensions of the component members in each drawing do not closely represent actual dimensions of the component members, a dimensional ratio of each component member, or the like.

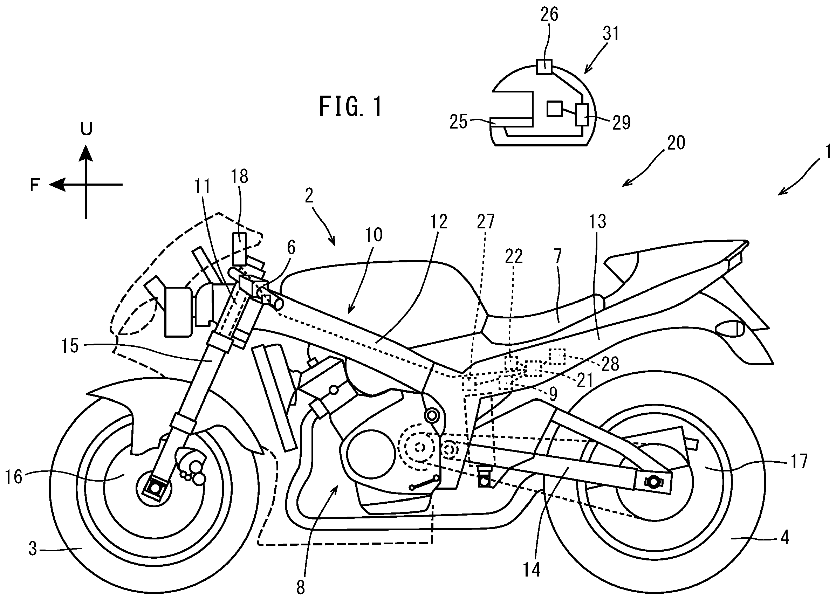

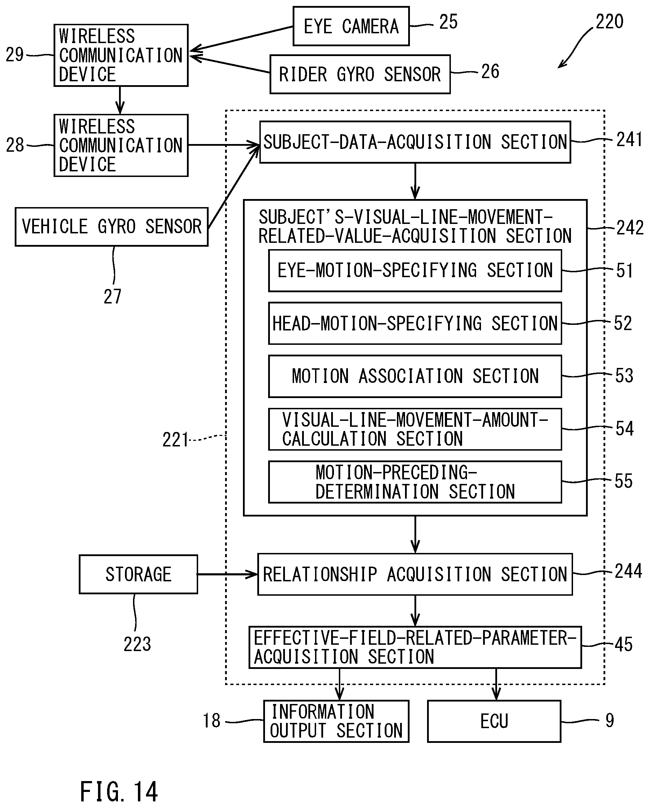

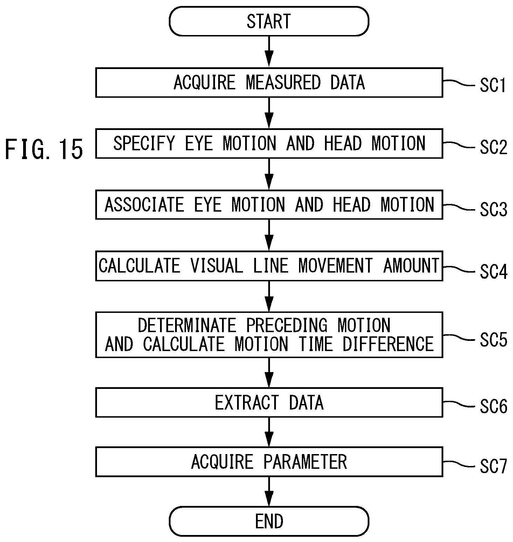

The following embodiments will be described using a motorcycle as an example of a transport apparatus. Therefore in the following description, an arrow F in FIG. 1 indicates a forward direction of the motorcycle. An arrow U in the drawings indicates an upward direction of the motorcycle. Also, a front and rear direction and a left and right direction mean a front and rear direction and a left and right direction when viewed from a rider who drives the motorcycle, respectively.
The third embodiment covered by the figures 14 and 15 is employed as example to explain the claimed subject-matter of the present invention, and other embodiments are employed as reference examples to support the understanding the present invention.

### [First Embodiment]

### <Entire Structure>

An overall structure of a vehicle 1 (a transport apparatus) including a visual-line-movement-related-value-acquisition device 20 according to a first embodiment of the present teaching is illustrated in FIG. 1. In this embodiment, the vehicle 1 is a motorcycle having a typical structure. Note that the vehicle 1 is not limited to the motorcycle and may be a vehicle, such as a three-wheeled vehicle, a four-wheeled vehicle, or the like.

The vehicle 1 includes a vehicle body 2, a front wheel 3, and a rear wheel 4. The vehicle body 2 supports each of components, such as a handle 6, a seat 7, a power unit 8, or the like. The vehicle body 2 includes a frame 10 and a pair of front forks 15. The frame 10 supports each of the components, such as the handle 6, the seat 7, the power unit 8, or the like.

The frame 10 includes a head pipe 11, a main frame 12, a seat rail 13, and a swing arm 14.

The head pipe 11 is located in a front portion of the vehicle 1 and rotatably supports a steering shaft (not illustrated) connected to the handle 6. The main frame 12 is connected to the head pipe 11 so as to extend toward a rear of the vehicle from the head pipe 11. The main frame 12 supports the power unit 8 or the like.

The pair of front forks 15 is connected to a lower end portion of the steering shaft. The front wheel 3 is rotatably supported at the lower end portions of the pair of front forks 15. Thus, the steering shaft and the pair of front forks 15 are caused to rotate together around the steering shaft as a center by operating the handle 6 and therefore, the front wheel 3 also rotates around the steering shaft as a center.

A front brake 16 is arranged between the lower end portions of the pair of front forks 15 and the front wheel 3. The front brake 16 is caused to operate, for example, by an operation of an unillustrated brake lever provided in the handle 6.

The seat rail 13 is connected to a rear portion of an upper portion of the main frame 12 so as to extend toward the rear of the vehicle. The seat rail 13 supports the seat 7.

The swing arm 14 is rotatably supported in an up-down direction at a rear portion of a lower portion of the main frame 12. The rear wheel 4 is rotatably supported at a rear end portion of the swing arm 14. A rear wheel brake 17 is arranged between the swing arm 14 and the rear wheel 4. The rear wheel brake 17 is caused to operate, for example, by an operation of a brake pedal (not illustrated).

Also, the vehicle 1 includes an electric control unit (ECU) 9, an information output section 18, and the visual-line-movement-related-value-acquisition device 20.

The ECU 9 controls each component to be controlled in the vehicle body 2. That is, the ECU 9 controls an operation of each component to be controlled in the vehicle body 2 using a control parameter. The ECU 9 is arranged under the seat 7 in the vehicle body 2. Note that, as will be described later, the ECU 9 controls the operation of each component to be controlled in the vehicle body 2 in accordance with an output of the visual-line-movement-related-value-acquisition device 20.

The information output section 18 transfers an output result of the visual-line-movement-related-value-acquisition device 20 to the rider who drives the vehicle 1. In this embodiment, the information output section 18 includes, for example, a display, an audio output device, or the like. Note that the vehicle 1 may include a device that transfers an output of the visual-line-movement-related-value-acquisition device 20 to the rider via vibration or the like.

The visual-line-movement-related-value-acquisition device 20 continuously acquires a same subject's visual line movement-related value (a subject's visual line movement-related value) related to the visual line movement of the rider (a subject) who drives the vehicle 1. The same subject's visual line movement-related value is a value related to an effective visual field of the rider (the subject), as will be described later. That is, the visual-line-movement-related-value-acquisition device 20 acquires a parameter related to the effective visual field of the rider (the subject).

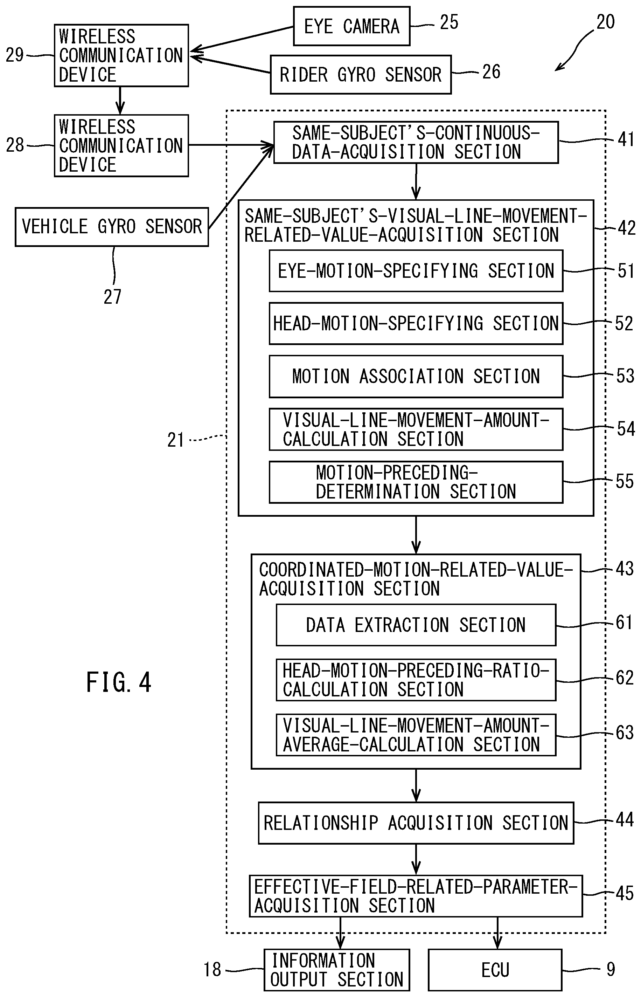

The visual-line-movement-related-value-acquisition device 20 includes an arithmetic processor 21, a storage 22, a vehicle gyro sensor 27, and a wireless communication device 28. The arithmetic processor 21 performs various arithmetic operations in the visual-line-movement-related-value-acquisition device 20. That is, the arithmetic processor 21 forms a portion of a functional block in FIG. 4, which will be described later. The storage 22 stores data acquired by the vehicle gyro sensor 27, and an eye camera 25 and a rider gyro sensor 26, which will be described later, is a result of an arithmetic operation performed by the arithmetic processor 21, or the like. The arithmetic processor 21, the storage 22, the vehicle gyro sensor 27, and the wireless communication device 28 are arranged under the seat 7 in the vehicle body 2.

Also, the visual-line-movement-related-value-acquisition device 20 includes the eye camera 25, the rider gyro sensor 26, and a wireless communication device 29, each of which is provided in a helmet 31 that the rider who drives the vehicle 1 wears.

The eye camera 25 is arranged in a lower portion of an opening formed in a front surface of the helmet 31. The rider gyro sensor 26 and the wireless communication device 29 are provided in the helmet 31.

A detailed structure of the visual-line-movement-related-value-acquisition device 20 will be described later.

### (Relationship between Visual Line Movement-related Value and effective visual field)

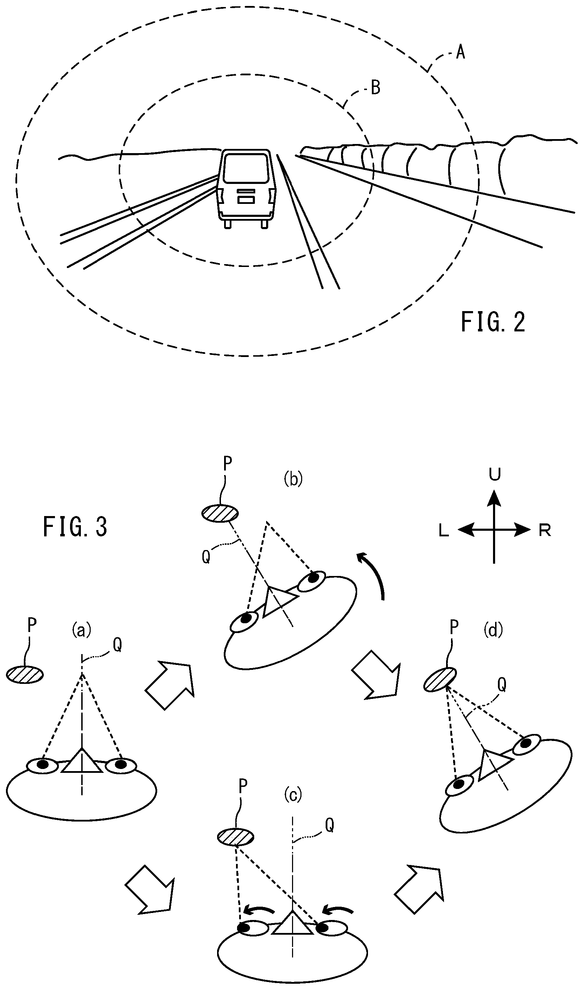

First, a relationship between the same subject's visual line movement-related value acquired by the visual-line-movement-related-value-acquisition device 20 and the effective visual field of the subject will be described. FIG. 2 is a view schematically illustrating the effective visual field of the rider who drives the vehicle 1. The effective visual field is a range in which the subject can collect visual information effectively used in an action of capturing a visual object in a field of vision.

An area of an effective visual field of a person changes in accordance with an internal state of the person. As used herein, the term "internal state" means an attention level (a state), an awake level (a state), a concentration level (a degree of concentration), a margin, information processing performance, or the like. Furthermore, the internal state is not limited to a mental state and also means a physical state (a fatigue level).

Specifically, the effective visual field of the person tends to be narrower as the internal state of the person becomes poorer. In FIG. 2, each of the reference signs A and B schematically denotes an effective visual field of a same person in accordance with internal states of the person. The effective visual field B is a range of a field of vision when the internal state is poorer than that of the effective visual field A. As illustrated in FIG. 2, the effective visual field B when the internal state is poorer, is narrower than the effective visual field A.

Therefore, there is a probability that the effective visual field of the person is detected, and thereby, the internal state of the person can be understood. This embodiment focuses on the visual line movement of the person in order to detect the effective visual field of the person.

People detect a visual object to be gazed at next by using information achieved from inside of the effective visual field and perform visual line movement by eye motion and head motion on the detected visual object. When the visual object is located inside the effective visual field, the eye motion precedes the head motion. On the other hand, when the visual object is located outside the effective visual field, the head motion precedes the eye motion. As described above, depending on whether or not the visual object is located inside the effective visual field, an action of viewing the visual object by people (which will be hereinafter referred to as a viewing action) differs.

Note that the viewing action includes at least one of motion of eyes (eye motion) and motion of a head (head motion). As used herein, the term "eye motion" means motion of eyes relative to a head. That is, the eye motion means the movement of the eyes relative to the head. The eye motion includes saccade (saccadic eye motion) in which, for example, an absolute value of an eye rotation angular velocity is a threshold α (for example, 30 deg/sec) or more, and an absolute value of a motion amount obtained by integrating the rotation angular velocity is a threshold β (for example, 3.5 deg) or more. The head motion is a motion of a head when the head moves or rotates in absolute coordinates in the up-down direction and the left-right direction. That is, the head motion includes motion of the head when the head moves or rotates relative to a body, motion of the head when the head and the body move or rotate together, and motion of the head when the head and the body separately move or rotate.

FIG. 3 is a view schematically illustrating an example of a viewing action. The person performs a viewing action so as to view a visual object P on a central line between left and right eyes in the left-right direction. Therefore, as illustrated in FIG. 3(a), when the visual object P is off a centerline Q (which will be hereinafter merely referred to as a centerline between the left and right eyes) located at an equal distance from each of the left eye and the right eye in the left-right direction, as illustrated in FIG. 3(d), the person views the visual object P on the centerline Q between the left and right eyes in the left-right direction by performing a viewing action illustrated in FIG. 3(b) or FIG. 3(c).

Note that, in the examples illustrated in FIG. 3, at least one of the eyes and the head of the person moves in order of (a), (b), and (d) or in order of (a), (c), and (d) over time. Also in FIG. 3, the visual object P, not in a cross-sectional view, is indicated by oblique lines for describing the corresponding viewing action.

As illustrated in FIG. 3(a), when the visual object P is off the centerline Q between the left and right eyes in the left-right direction, the person moves the eyes and the head to view the visual object P on the centerline Q between the left and right eyes.

FIG. 3(b) schematically illustrates a state in which head motion occurs first. In this case, in the left-right direction, movement of the head is started such that the visual object P is located on the centerline Q between the left and right eyes. Thereafter, the person starts moving the left and right eyes and views the visual object P (FIG. 3(d)). As illustrated in FIG. 3(b), when the person views the visual object P with the left and right eyes, the person starts the head motion first in some cases. As described above, a viewing action in which a start of the head motion precedes a start of the eye motion in time is referred to as a "head preceding type viewing action" or merely as a "head preceding type."

FIG. 3(c) schematically illustrates a state in which eye motion occurs first. In this case, in the left-right direction, movement of the eyes is started such that the visual object P is located on the centerline Q between the left and right eyes. Thereafter, the person starts moving the head (FIG. 3(d)). As illustrated in FIG. 3(c), when the person views the visual object P with the left and right eyes, the person starts the eye motion first in some cases. As described above, a viewing action in which a start of eye motion precedes a start of head motion in time will be referred to as an "eye preceding type viewing action" or merely as an "eye preceding type."

As described above, in a case in which the visual object P is off the centerline between the left and right eyes in the left-right direction, when the person views the visual object **P,** there is the head preceding type viewing action and the eye preceding type viewing action as viewing actions.

The present inventors presumed that the eye preceding type viewing action tends to occur when a visual object is located inside an effective visual field and the head preceding type viewing action tends to occur when a visual object is not located inside the effective visual field. Based on the presumption, the present inventors considered that, as the effective visual field becomes narrower, a visual object is less likely to be located inside the effective visual field, and therefore, the head preceding type viewing action tends to occur in many cases.

As described above, it is considered that, when the person performs the viewing action to view the visual object on the central line between the left and right eyes, whether the viewing action is a head preceding type viewing action or an eye preceding type viewing action is influenced by the effective visual field of the person. Then, as described above, the field of vision of the person is influenced by an internal state of the person. Therefore, it is considered that the effective visual field related to the internal state of the person can be relatively evaluated by analyzing the head preceding type viewing action and the eye preceding type viewing action in the viewing action.

However, change of the internal state of the person largely differs among individuals. Moreover, the internal state of the person largely changes in accordance with the situation in which the person is put. Therefore, even when the effective visual field indicating the internal state of the person can be relatively evaluated by analyzing the head preceding type viewing action in the viewing action in the above-described manner, it is difficult to detect the effective visual field that changes in accordance with the internal state.

**In** contrast, the present inventors noticed through further examinations on influences of individual differences on change of the internal state of the person that continuous data is acquired from a same subject and thereby, data in consideration of individual characteristics of the same subject can be acquired. Thus, the effective visual field can be detected while taking influences of individual differences on changes of the internal state into consideration.

Based on the above-described findings, the visual-line-movement-related-value-acquisition device 20 according to this embodiment is configured to obtain a relationship between a visual line movement amount as a visual line movement-related value related to a visual line movement and a ratio at which head motion precedes (the head motion preceding ratio) using data continuously acquired from the same subject. Then, the visual-line-movement-related-value-acquisition device 20 outputs a value related to the effective visual field from the above-described relationship.

A specific structure of the visual-line-movement-related-value-acquisition device 20 will be described below.

### (Visual-Line-Movement-related-Value-Acquisition Device)

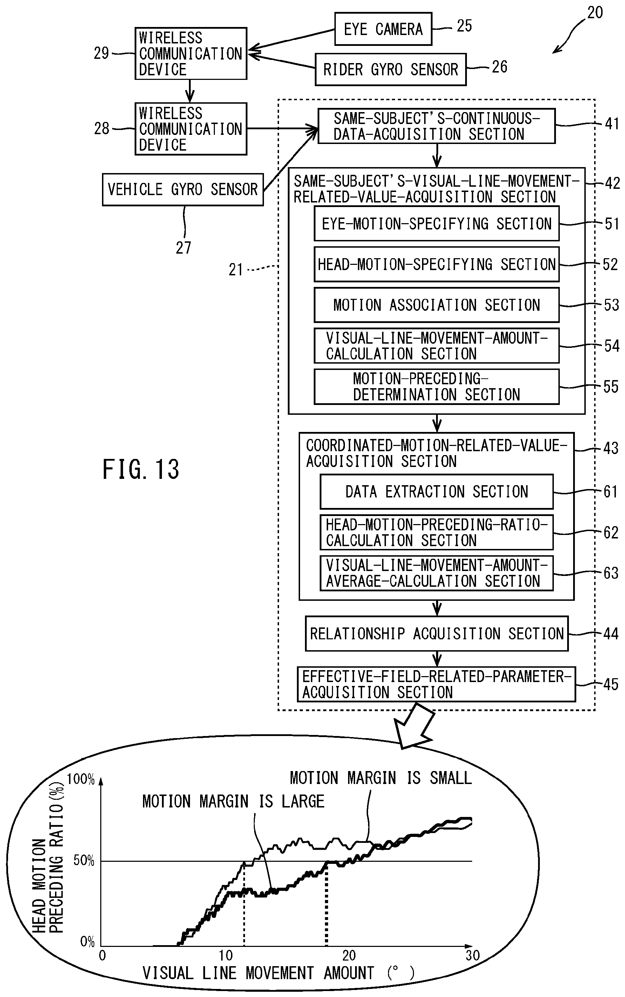

FIG. 4 and FIG. 13 are functional block diagrams each illustrating a structure of the visual-line-movement-related-value-acquisition device 20.

The visual-line-movement-related-value-acquisition device 20 includes the arithmetic processor 21, the storage 22, the eye camera 25, the rider gyro sensor 26 (a head-motion-measurement section), the vehicle gyro sensor 27 (a transport-apparatus-motion-measurement section), the wireless communication device 28, and the wireless communication device 29. Note that, in FIG. 4, illustration of the storage 22 is omitted.

The eye camera 25 measures eye motion of the rider (the subject) who wears the helmet 31. A measurement result of the eye camera 25 is transmitted to the arithmetic processor 21 via the wireless communication devices 28 and 29.

The rider gyro sensor 26 measures head motion of the rider (the subject) who wears the helmet 31. Note that, in this embodiment, motion of the helmet 31 is assumed to be the head motion of the rider. A measurement result of the rider gyro sensor 26 is transmitted to the arithmetic processor 21 via the wireless communication devices 28 and 29.

The vehicle gyro sensor 27 measures a behavior of the vehicle 1, such as turning, an inclination in the front-rear direction, an inclination in the left-right direction, or the like. A measurement result of the vehicle gyro sensor 27 is input to the arithmetic processor 21.

The arithmetic processor 21 acquires and outputs the visual line movement-related value related to the visual line movement of the rider (the subject), based on each of measurement results of the eye camera 25, the rider gyro sensor 26, and the vehicle gyro sensor 27. Specifically, the arithmetic processor 21 includes a same-subject's-continuous-data-acquisition section 41, a same subject's-visual-line-movement-related-value-acquisition section 42 (the subject's-visual-line-movement-related-value-acquisition section), a coordinated-motion-related-value-acquisition section 43, a relationship acquisition section 44, and an effective-visual-field-related-parameter-acquisition section (the subject's-visual-line-movement-related-value-output section) 45.

The same-subject's-continuous-data-acquisition section 41 acquires each of measurement results output from the eye camera 25, the rider gyro sensor 26, and the vehicle gyro sensor 27 as same subject's continuous data. The same subject's continuous data includes eye motion data acquired by continuously measuring the eye motion of the same subject by the eye camera 25 and head motion data acquired by continuously measuring the head motion of the same subject by the rider gyro sensor 26 and the vehicle gyro sensor 27. The head motion data can be acquired by deducting a measured value (vehicle behavior data) of the vehicle gyro sensor 27 from a measured value of the rider gyro sensor 26. Thus, influences of the behavior of the vehicle 1 on the head motion data of the rider (the subject) can be prevented.

The same subject's-visual-line-movement-related-value-acquisition section 42 acquires the same subject's visual line movement-related value (the subject's visual line movement-related value) that is a value related to the visual line movement of the rider (the subject), based on the same subject's continuous data acquired by the same-subject's-continuous-data-acquisition section 41. Specifically, the same subject's-visual-line-movement-related-value-acquisition section 42 continuously calculates an eye movement amount in the eye motion data of the same subject's continuous data, continuously calculates a head movement amount of the head motion data of the same subject's continuous data corresponding to the eye motion data, and continuously obtains a sum of the eye movement amount and the head movement amount. Also, the same subject's-visual-line-movement-related-value-acquisition section 42 specifies a start timing of the head motion of the subject and a start timing of the eye motion of the subject and determines which one of the head motion or the eye motion precedes, based on the specified timings.

Herein, the visual line movement amount of the subject is represented by the total of the eye motion amount and the head motion amount of the subject. That is, in this embodiment, the sum of the eye movement amount and the head movement amount is the visual line movement amount of the subject. Therefore, the same subject's-visual-line-movement-related-value-acquisition section 42 continuously obtains the visual line movement amount of the subject.

More specifically, the same subject's-visual-line-movement-related-value-acquisition section 42 includes an eye-motion-specifying section 51, a head-motion-specifying section 52, a motion association section 53, a visual-line-movement-amount-calculation section 54, and a motion-preceding-determination section 55.

The eye-motion-specifying section 51 specifies a zone of the eye motion from the eye motion data (for example, the eye rotation angular velocity) of the same subject's continuous data acquired by the same-subject's-continuous-data-acquisition section 41 and specifies the start timing of the eye motion. Specifically, for example, when the absolute value of the eye rotation angular velocity is the threshold α (for example, 30 deg/sec) or more and the absolute value of the motion amount obtained by integrating the rotation angular velocity is the threshold β (for example, 3.5 deg) or more, the eye-motion-specifying section 51 specifies the eye motion from the eye motion data. This eye motion is called saccadic eye motion or rapid eye motion.

Also, the eye-motion-specifying section 51 determines a time point at which the absolute value of the rotation angular velocity exceeds the threshold α for the first time in the detected eye motion to be a start of the eye motion.

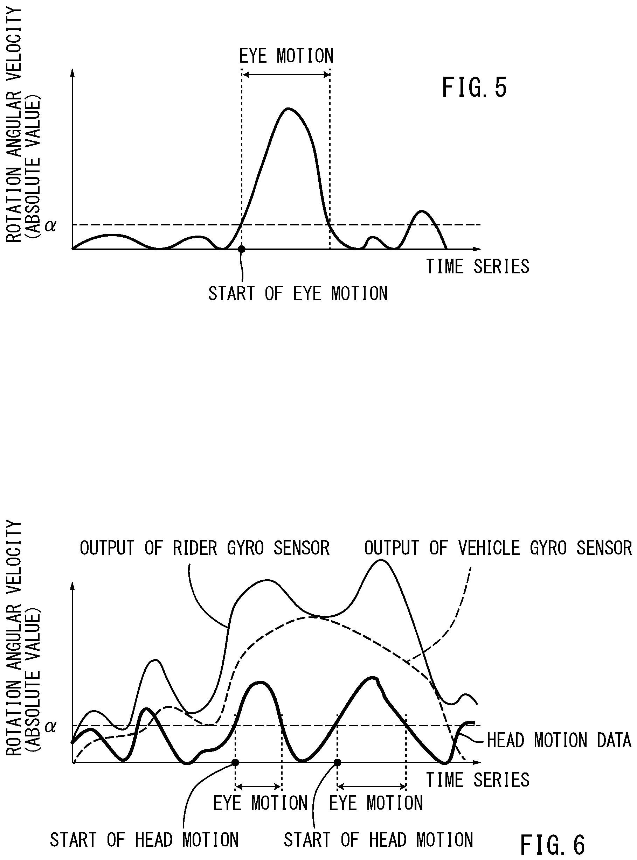

FIG. 5 is a graph illustrating an example of time change of the eye rotation angular velocity. In the example of FIG. 5, in other ranges than a range indicated as "EYE MOTION," the absolute value of the eye rotation angular velocity is less than the threshold α, or the absolute value of the eye motion amount is less than the threshold β, and therefore, it is not specified as eye motion.

The head-motion-specifying section 52 specifies a zone of the head motion from the head motion data (for example, the head rotation angular velocity) of the same subject's continuous data acquired by the same-subject's-continuous-data-acquisition section 41 and a start timing of the head motion. Specifically, for example, when the absolute value of the head rotation angular velocity is a threshold γ (for example, 10 deg/sec) or more and the absolute value of the motion amount obtained by integrating the rotation angular velocity is a threshold δ (for example, 3.5 deg) more, the head-motion-specifying section 52 specifies head motion from the head motion data.

Also, the head-motion-specifying section 52 determines a time point at which the absolute value of the rotation angular velocity exceeds the threshold γ for the first time in the detected head motion to be a start of the head motion.

FIG. 6 is a graph illustrating an example of time change of the head rotation angular velocity. In the example of FIG. 6, in other ranges than a range indicated as "HEAD MOTION," the absolute value of the head rotation angular velocity is less than the threshold γ or the absolute value of the head motion amount is less than the threshold δ, and therefore, the head motion is not specified. Note that, in FIG. 6, not only the head motion data of the rider (the subject) but also a measured value (output) of the rider gyro sensor 26 and a measured value (output) of the vehicle gyro sensor 27 are illustrated.

The motion association section 53 associates the eye motion detected by the eye-motion-specifying section 51 and the head motion detected by the head-motion-specifying section 52 at the timings at which the motions are specified.

The visual-line-movement-amount-calculation section 54 calculates, as a visual line movement amount, a total of the motion amount (an integrated value of the rotation angular velocity) in a zone in which each motion is specified in the eye motion and the head motion associated with each other by the motion association section 53.

The motion-preceding-determination section 55 determines preceding motion of the eye motion and the head motion associated with each other by the motion association section 53.

Specifically, when there is not motion specified to be the head motion by the head-motion-specifying section 52 in the zone in which the eye motion is specified by the eye-motion-specifying section 51, the motion-preceding-determination section 55 determines that the eye motion precedes. Also, even in a case in which there is motion specified to be the head motion by the head-motion-specifying section 52 in the zone in which the eye motion is specified by the eye-motion-specifying section 51, when the start of the eye motion is earlier than the start of the head motion, the motion-preceding-determination section 55 determines that the eye motion precedes.

On the other hand, in a case in which there is motion specified to be the eye motion by the eye-motion-specifying section 51 in the zone in which the head motion is specified by the head-motion-specifying section 52, when the start of the head motion is earlier than the start of the eye motion, the motion-preceding-determination section 55 determines that the head motion precedes. Note that, when there is not the zone in which the eye motion is specified in the zone in which the head motion is specified, a state in which the subject looks at one point (a state in which the subject gazes at one point or stops) is determined, and therefore, a viewing action is not determined.

A determination result of the motion-preceding-determination section 55 is linked to the visual line movement amount calculated by the visual-line-movement-amount-calculation section 54 and is output to the coordinated-motion-related-value-acquisition section 43. That is, the visual line movement amount is output to the coordinated-motion-related-value-acquisition section 43 with information (preceding motion information) on which one of the head motion or the eye motion is preceding motion.

The coordinated-motion-related-value-acquisition section 43 acquires a coordinated motion-related value related to a coordinated motion of the eye motion and the head motion of the subject, based on data output from the same subject's-visual-line-movement-related-value-acquisition section 42. Note that, in this embodiment, among the data output from the same subject's-visual-line-movement-related-value-acquisition section 42, the visual line movement amount is the same subject's visual line movement-related value. Also, the coordinated motion-related value is a value related to the coordinated motion of the eye motion and the head motion of the same subject and is the head motion preceding ratio in this embodiment.

Specifically, the coordinated-motion-related-value-acquisition section 43 includes a data extraction section 61, a head-motion-preceding-ratio-calculation section 62, and a visual-line-movement-amount-average-calculation section 63.

The data extraction section 61 extracts, among data output from the same subject's-visual-line-movement-related-value-acquisition section 42, that is, data in which the preceding motion information and the visual line movement amount are linked to each other, acquired data that has been acquired in a predetermined period, and arranges the extracted acquired data in an order of magnitude of the visual line movement amount. Thereafter, the data extraction section 61 extracts a predetermined number of continuous data (arithmetic operation target data) from the acquired data arranged in the order of the magnitude of the visual line movement amount.

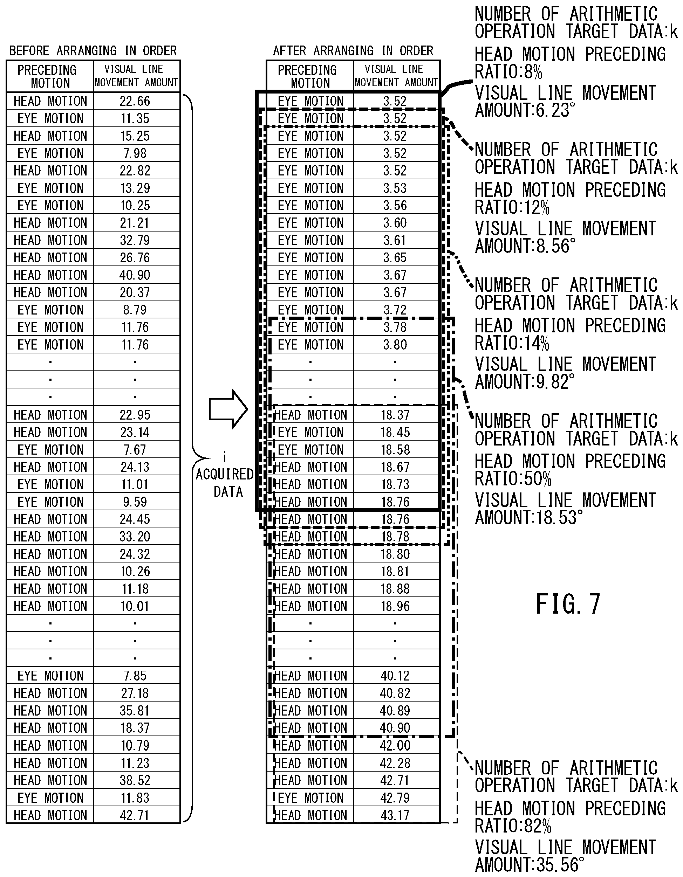

**In** a left side of FIG. 7, among the data in which the preceding motion information (indicated merely as PRECEDING MOTION" in FIG. 7) and the visual line movement amount are linked to each other, an example of the acquired data acquired in the predetermined period is illustrated. Note that, in the example of FIG. 7, i data acquired in the predetermined period are extracted as acquired data. In a right side of FIG. 7, how the extracted i acquired data are arranged in an ascending order of the visual line movement amount and a predetermined number (k in this embodiment) of arithmetic operation target data are extracted from the extracted i acquired data is illustrated.

The head-motion-preceding-ratio-calculation section 62 calculates a ratio of data in which head motion precedes in the predetermined number of acquired data extracted by the data extraction section 61, that is, the head motion preceding ratio (a representative value). The head motion preceding ratio is obtained by obtaining the ratio of the number of data in which head motion precedes to the predetermined number of extracted arithmetic operation target data.

The visual-line-movement-amount-average-calculation section 63 calculates an average value (the representative value) of the visual line movement amounts in the predetermined number of arithmetic operation target data which have been extracted by the data extraction section 61 and for which the head motion preceding ratio has been calculated by the head-motion-preceding-ratio-calculation section 62.

The head motion preceding ratio calculated by the head-motion-preceding-ratio-calculation section 62 and the average value of the visual line movement amounts calculated by the visual-line-movement-amount-average-calculation section 63 are linked to each other and are thus output.

Note that, after the head-motion-preceding-ratio-calculation section 62 calculates the head motion preceding ratio, and the visual-line-movement-amount-average-calculation section 63 calculates the average value of the visual line movement amounts, the data extraction section 61 extracts a predetermined number of arithmetic operation target data from next data for head data of the extracted predetermined number of arithmetic operation target data. The head-motion-preceding-ratio-calculation section 62 and the visual-line-movement-amount-average-calculation section 63 calculate the head motion preceding ratio and an average value of the visual line movement amounts, respectively, each time the predetermined number of arithmetic operation target data are extracted by the data extraction section 61.

Specifically, as illustrated in the right side of FIG. 7, the predetermined number of the arithmetic operation target data are extracted in the ascending order of the visual line movement amount from data in which the visual line movement amount is smallest among the i acquired data, the head-motion-preceding-ratio-calculation section 62 calculates the head motion preceding ratio, and the visual-line-movement-amount-average-calculation section 63 calculates the average value of the visual line movement amounts. Thereafter, the predetermined number of the arithmetic operation target data are extracted in the ascending order of the visual line movement amount from data in which the visual line movement amount is the second smallest among the i acquired data, the head-motion-preceding-ratio-calculation section 62 calculates the head motion preceding ratio, and the visual-line-movement-amount-average-calculation section 63 calculates the average value of the visual line movement amounts. The data extraction section 61 repeats the above-described operation until calculation of the head motion preceding ratio by the head-motion-preceding-ratio-calculation section 62 and calculation of the average value of the visual line movement amounts by the visual-line-movement-amount-average-calculation section 63 are completed for all of the i acquired data.

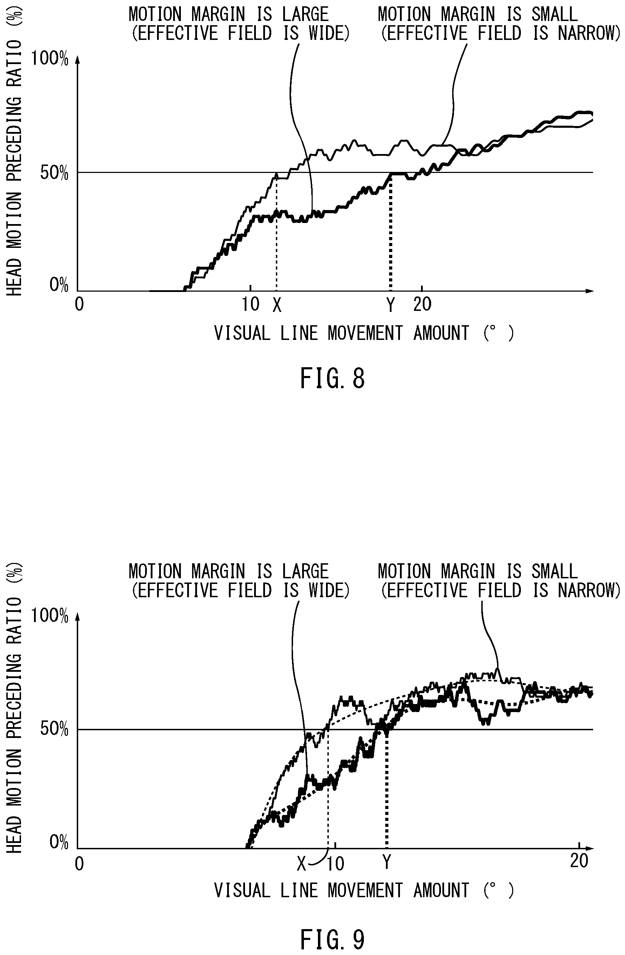

The relationship acquisition section 44 generates data (for example, a graph, a table, or the like) indicating a relationship between the head motion preceding ratio and the visual line movement amount using the head motion preceding ratio and the average value of the visual line movement amounts output from the coordinated-motion-related-value-acquisition section 43. As an example of the data, the relationship between the head motion preceding ratio and the visual line movement amount is illustrated in a graph in FIG. 8.

In FIG. 8, the relationship between the head motion preceding ratio and the visual line movement amount when a driving margin of the rider who drives the vehicle 1 is large is indicated by a thick line. On the other hand, in FIG. 8, the relationship between the head motion preceding ratio and the visual line movement amount when the driving margin of the rider who drives the vehicle 1 is small is indicated by a thin line. Note that, when the driving margin of the rider who drives the vehicle 1 is large, the internal state of the rider is good. On the other hand, when the driving margin of the rider who drives the vehicle 1 is small, the internal state of the rider is poor.

As illustrated in FIG. 8, in a range in which the head motion preceding ratio is 40% to 60%, differences in the visual line movement amount are remarkable due to differences in the driving margin of the rider, that is, differences in the internal state of the rider. Specifically, when the head motion preceding ratio is 50%, the differences in the visual line movement amount are more remarkable.

Incidentally, as described above, when the visual object is located outside of the effective visual field of the person, the head motion precedes. That is, when the internal state becomes poorer and the field of vision of the person becomes narrower, the head motion precedes. Therefore, the visual line movement amount when the head motion preceding ratio is a predetermined value is evaluated by using FIG. 8, and thereby, the field of vision of the person can be detected. Note that the predetermined value may be any value as long as the value is a value at which the visual line movement amount differs in accordance with the internal state, but preferably, for example, 40% to 60%, and particularly 50% is preferable.

The effective-visual-field-related-parameter-acquisition section 45 acquires, as the parameter related to the effective visual field, a visual line movement amount X, Y when the head motion preceding ratio is a predetermined value (for example, 50%) in FIG. 8 using characteristics of FIG. 8 described above. Specifically, in FIG. 8, in a case in which the driving margin is large, the visual line movement amount when the head motion preceding ratio is the predetermined value is Y. The visual line movement amount Y is larger than the visual line movement amount X when the driving margin is small. Therefore, with the same head motion preceding ratio, the effective visual field is larger when the driving margin is large than when the driving margin is small.

Thus, the effective visual field of the person can be detected using the parameter acquired by the effective-visual-field-related-parameter-acquisition section 45. Note that the visual line movement amount X, Y acquired as the parameter related to the effective visual field is the same subject's visual line movement-related value (the subject visual line movement-related value) related to the visual line movement of the subject.

Note that, in this embodiment, the effective-visual-field-related-parameter-acquisition section 45 acquires the visual line movement amount X, Y when the head motion preceding ratio is 50% in the relationship between the head motion preceding ratio and the visual line movement amount illustrated in FIG. 8. However, the predetermined value may be a value between 40% and 60%. That is, the effective-visual-field-related-parameter-acquisition section 45 may be configured to use any value as the predetermined value as long as the value is a value of the head motion preceding ratio with which a difference in the visual line movement amount can be detected in accordance with the internal state of the person in the relationship between the head motion preceding ratio and the visual line movement amount.

The effective-visual-field-related-parameter-acquisition section 45 may be configured to, when there are a plurality of visual line movement amounts that correspond to the predetermined value of the head motion preceding ratio in the data indicating the relationship between the head motion preceding ratio and the visual line movement amount illustrated in FIG. 8, acquire a value at which the visual line movement amount is the smallest as the parameter related to the effective visual field and also acquire a value at which the visual line movement amount is the largest as the parameter related to the effective visual field. Also, the effective-visual-field-related-parameter-acquisition section 45 may be configured to, when there are a plurality of visual line movement amounts that correspond to the predetermined value of the head motion preceding ratio as described above, acquire an intermediate value of the value at which the visual line movement amount is the smallest and the value at which the visual line movement amount is the largest as the parameter related to the effective visual field and also acquire an average value of the visual line movement amounts as the parameter related to the effective visual field.

Also, as illustrated in FIG. 9, the visual line movement amounts X and Y corresponding to the predetermined values of the head motion preceding ratio on an approximate curve of data indicating the relationship between the head motion preceding ratio and the visual line movement amount may be acquired as the parameters related to the effective visual field.

The effective-visual-field-related-parameter-acquisition section 45 outputs the visual line movement amount as the parameter related to the effective visual field to the ECU 9 and the information output section 18. In accordance with the value output from the effective-visual-field-related-parameter-acquisition section 45, the ECU 9 controls each component of the vehicle 1 to be controlled in a manner suitable for the internal state of the rider who drives the vehicle 1. The information output section 18 informs the rider of information related to the internal state in accordance with the value output from the effective-visual-field-related-parameter-acquisition section 45.

### (Operation of Visual-Line-Movement-Related-Value-Acquisition Device)

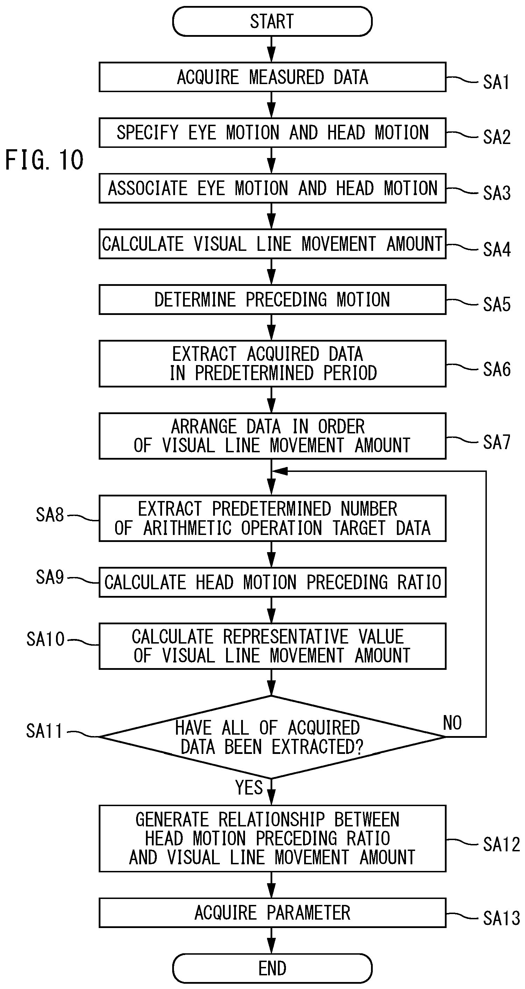

Next, an operation of the visual-line-movement-related-value-acquisition device 20 will be described. FIG. 10 illustrates a flowchart of the operation of the visual-line-movement-related-value-acquisition device 20.

When a flow illustrated in FIG. 10 starts, in Step SA1, the same-subject's-continuous-data-acquisition section 41 acquires measured data continuously measured from each of the eye camera 25, the rider gyro sensor 26, and the vehicle gyro sensor 27. Specifically, the same-subject's-continuous-data-acquisition section 41 continuously acquires eye motion data related to eye motion of a same rider (a subject), the eye motion data having been measured by the eye camera 25. Also, the same-subject's-continuous-data-acquisition section 41 continuously acquires head motion data related to head motion of the same rider (the subject), the head motion data having been measured by the rider gyro sensor 26. Also, the same-subject's-continuous-data-acquisition section 41 continuously acquires vehicle behavior data related to a behavior of the vehicle 1, the vehicle behavior data having been measured by the vehicle gyro sensor 27.

Next, in Step SA2, the eye-motion-specifying section 51 of the same subject's-visual-line-movement-related-value-acquisition section 42 specifies eye motion, based on the eye motion data acquired by the same-subject's-continuous-data-acquisition section 41, and also specifies a start timing of the eye motion. Specifically, for example, when an absolute value of an eye rotation angular velocity is the threshold α or more and an absolute value of a motion amount obtained by integrating the rotation angular velocity is the threshold β or more, the eye-motion-specifying section 51 specifies the eye motion from the eye motion data. Also, the eye-motion-specifying section 51 determines, as a start of the eye motion, a time point at which the absolute value of the rotation angular velocity exceeds the threshold α for the first time in the detected eye motion.

In Step SA2, the head-motion-specifying section 52 of the same subject's-visual-line-movement-related-value-acquisition section 42 specifies head motion, based on the head motion data acquired by the same-subject's-continuous-data-acquisition section 41, and also specifies a start timing of the head motion. Note that the head motion data is obtained by deducting a measured value of the vehicle gyro sensor 27 (vehicle behavior data) from a measured value of the rider gyro sensor 26.

Specifically, for example, when an absolute value of a rotation angular velocity of the head is the threshold γ or more and an absolute value of a motion amount obtained by integrating the rotation angular velocity is the threshold δ or more, the head-motion-specifying section 52 specifies the head motion from the head motion data. Also, the head-motion-specifying section 52 determines, as a start of the head motion, a time point at which the absolute value of the rotation angular velocity exceeds the threshold γ for the first time in the detected head motion.

In subsequent Step SA3, the motion association section 53 of the same subject's-visual-line-movement-related-value-acquisition section 42 associates the eye motion detected by the eye-motion-specifying section 51 and the head motion detected by the head-motion-specifying section 52 with each other at the timings at which the motions are specified.

In Step SA4, the visual-line-movement-amount-calculation section 54 of the same subject's-visual-line-movement-related-value-acquisition section 42 calculates, as a visual line movement amount, the total of the motion amounts (an integrated value of the rotation angular velocity) in a zone in which each motion is specified in the eye motion and the head motion associated with each other by the motion association section 53.

In Step SA5, the motion-preceding-determination section 55 of the same subject's-visual-line-movement-related-value-acquisition section 42 determines preceding motion of the eye motion and the head motion associated with each other by the motion association section 53.

Specifically, when there is not motion specified to be the head motion by the head-motion-specifying section 52 in the zone in which the eye motion is specified by the eye-motion-specifying section 51, the motion-preceding-determination section 55 determines that the eye motion precedes. Also, even in a case in which there is motion specified to be the head motion by the head-motion-specifying section 52 in the zone in which the eye motion is specified by the eye-motion-specifying section 51, when the start of the eye motion is earlier than the start of the head motion, the motion-preceding-determination section 55 determines that the eye motion precedes.

On the other hand, in a case in which there is motion specified to be the eye motion by the eye-motion-specifying section 51 in the zone in which the head motion is specified by the head-motion-specifying section 52, when the start of the head motion is earlier than the start of the eye motion, the motion-preceding-determination section 55 determines that the head motion precedes. Note that, when there is not the zone in which the eye motion is specified in the zone in which the head motion is specified, a state in which the subject looks at one point (a state in which the subject gazes at one point or stops) is determined, and therefore, a viewing action is not determined.

A determination result (preceding motion information) of the motion-preceding-determination section 55 is linked to the visual line movement amount calculated by the visual-line-movement-amount-calculation section 54.

In Step SA6, the data extraction section 61 of the coordinated-motion-related-value-acquisition section 43 extracts acquired data that has been acquired in a predetermined period from data output from the same subject's-visual-line-movement-related-value-acquisition section 42, that is, data in which the preceding motion information and the visual line movement amount are linked to each other. Then, in Step SA7, the data extraction section 61 arranges the extracted acquired data in an order of magnitude of the visual line movement amount and extracts, in subsequent Step SA8, a predetermined number of continuous arithmetic operation target data from the continuously arranged acquired data.

In Step SA9, the head-motion-preceding-ratio-calculation section 62 of the coordinated-motion-related-value-acquisition section 43 calculates a ratio of data in which the head motion precedes in the predetermined number of arithmetic operation target data extracted by the data extraction section 61, that is, the head motion preceding ratio (the representative value).

In Step SA10, the visual-line-movement-amount-average-calculation section 63 of the coordinated-motion-related-value-acquisition section 43 calculates an average value (the representative value) of the visual line movement amounts in the predetermined number of arithmetic operation target data which have been extracted by the data extraction section 61 and for which the head motion preceding ratio has been calculated by the head-motion-preceding-ratio-calculation section 62.

In Step SA11, the data extraction section 61 determines whether all of the acquired data in the predetermined period have been extracted as the arithmetic operation target data. In Step SA11, when it is determined that all of the acquired data in the predetermined period have been extracted as the arithmetic operation target data (YES), the process proceeds to Step SA12 and subsequent steps. On the other hand, in Step SA11, when it is determined that all of the acquired data in the predetermined period have not been extracted as the arithmetic operation target data (NO), the process returns to Step SA8 and the data extraction section 61 extracts the predetermined number of arithmetic operation target data from the acquired data in the predetermined period. Note that, when the data extraction section 61 performs extraction of the predetermined number of arithmetic operation target data from the acquired data in the predetermined period, the data extraction section 61 extracts a predetermined number of arithmetic operation target data from next data for head data of the predetermined number of arithmetic operation target data that have been extracted.

In Step SA12 to which the process proceeds when it is determined in Step S11 that all of the acquired data in the predetermined period have been extracted as the arithmetic operation target data, the relationship acquisition section 44 generates a relationship between the head motion preceding ratio and the visual line movement amount (for example, FIG. 7). Thereafter, in Step SA13, the effective-visual-field-related-parameter-acquisition section 45 acquires a parameter related to the effective visual field of the rider (the subject) from the relationship between the head motion preceding ratio and the visual line movement amount. Specifically, the effective-visual-field-related-parameter-acquisition section 45 acquires, as the parameter related to the effective visual field, the visual line movement amount corresponding to a predetermined value (for example, 50%) of the head motion preceding ratio. Note that the predetermined value is a value of the head motion preceding ratio with which a difference in the visual line movement mount can be detected in accordance with the internal state of the person in the relationship between the head motion preceding ratio and the visual line movement amount.

Herein, Step SA1 is a same subject's continuous data acquisition step in which same subject's continuous data including a plurality of data related to the same subject is acquired and Step SA4 is a same subject's visual line movement-related value acquisition step in which a same subject's visual line movement-related value related to the visual line movement of the subject is acquired from the same subject's continuous data.

Step SA9 is a coordinated motion-related value acquisition step in which a coordinated motion-related value related to a coordinated motion of eye motion and head motion of the subject for the acquired subject's visual line movement-related value is acquired.

Furthermore, Step SA13 is a same subject's visual line movement-related value output step in which the same subject's visual line movement-related value related to the visual line movement of the subject when the coordinated motion-related value related to the coordinated motion of the eye motion and the head motion of the subject is a predetermined value is output from a relationship between the acquired same subject's visual line movement-related value related to the visual line movement of the subject and the acquired coordinated motion-related value related to the coordinated motion of the eye motion and the head motion of the subject.

Step SA7 is an arrangement step in which the acquired coordinated motion-related values related to the coordinated motion of the eye motion and the head motion of the subject for the same subject's visual line movement-related value related to the visual line movement of the subject are arranged in an order of magnitude of the acquired same subject's visual line movement-related value related to the visual line movement of the subject.

Steps AS9 and SA10 are representative value calculation steps in which a predetermined number of consecutive values are extracted from the coordinated motion-related values related to the coordinated motion of the eye motion and head motion of the subject, the coordinated motion-related values being arranged in the order, and a representative value of the consecutive values is calculated, and also, a representative value of the same subject's visual line movement-related values is calculated from the same subject's visual line movement-related values related to the visual line movement of the subject, the same subject's visual line movement-related values corresponding to the extracted coordinated motion-related values related to the coordinated motion of the eye motion and the head motion of the subject.

Furthermore, Step SA12 is a relationship generation step in which the relationship between the same subject's visual line movement-related value related to the visual line movement of the subject and the coordinated motion-related value related to the coordinated motion of the eye motion and the head motion of the subject is acquired from the calculated representative value of the coordinated motion-related values related to the coordinated motion of the eye motion and the head motion of the subject and the calculated representative value of the same subject's visual line movement-related values related to the visual line movement of the subject.

In this embodiment of the present teaching, the arithmetic processor 21 executes Steps SA1 to SA13 illustrated in FIG. 10. The visual-line-movement-related-value-acquisition device 20 and a visual line movement-related value acquisition method in this embodiment can be realized by execution of processing of Steps SA1 to SA13 by the arithmetic processor 21. In this case, the arithmetic processor 21 is formed of, for example, a processor.

In this embodiment, data and an arithmetic operation result obtained by the arithmetic processor 21 are stored in the storage 22. When the arithmetic processor 21 performs an arithmetic operation using the data stored in the storage 22, the arithmetic processor 21 reads the data and performs arithmetic processing. The storage 22 is formed of a storage medium including a storage device, such as a memory, a hard disk, or the like.

In the structure of this embodiment, the visual-line-movement-related-value-acquisition device 20 continuously acquires the same subject's continuous data including a plurality of data related to the same rider (the subject) and acquires the visual line movement amount as the same subject's visual line movement-related value and the head motion preceding ratio as the coordinated motion-related value using the same subject's continuous data. Then, the visual-line-movement-related-value-acquisition device 20 outputs the visual line movement amount when the head motion preceding ratio is the predetermined value from a relationship between the visual line movement amount and the head motion preceding ratio.

Thus, the same subject's visual line movement-related value and the coordinated motion-related value less influenced by individual differences of the subject can be acquired. Furthermore, in the relationship between the same subject's visual line movement-related value and the coordinated motion-related value, an index related to the visual line movement of the subject can be obtained, without being influenced by individual differences, by outputting the same subject's visual line movement-related value when the coordinated motion-related value is the predetermined value. Then, an index related to the effective visual field of the subject can be obtained from the index related to the visual line movement of the subject.

The parameter related to the effective visual field acquired by the visual-line-movement-related-value-acquisition device 20 having the structure of this embodiment can be used when a subject exercises or performs an operation of a machine. That is, when the subject exercises or performs the operation of the machine, the parameter related to the effective visual field is acquired by the visual-line-movement-related-value-acquisition device 20, and thereby, the acquired parameter can be used in determination of the internal state of the subject when the subject exercises or performs the operation of the machine. Also, the acquired parameter can be used in relative evaluation between subjects or the like for the internal state when the subject exercises or performs the operation of the machine. Furthermore, the acquired parameter can be also used in evaluation and management of the internal state of the subject in an operation and a work in a virtual space.

As used herein, the term "internal state" means an attention level (a state), an awake level (a state), a concentration level (a degree of concentration), a margin, information processing performance, or the like. Furthermore, the internal state is not limited to a mental state and also means a physical state (a fatigue level).

### [Second Embodiment]

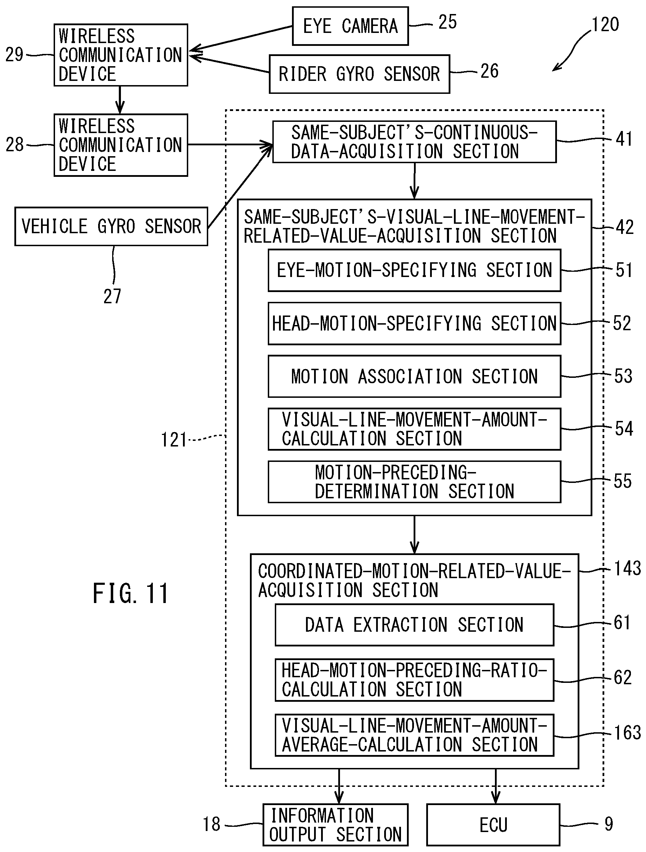

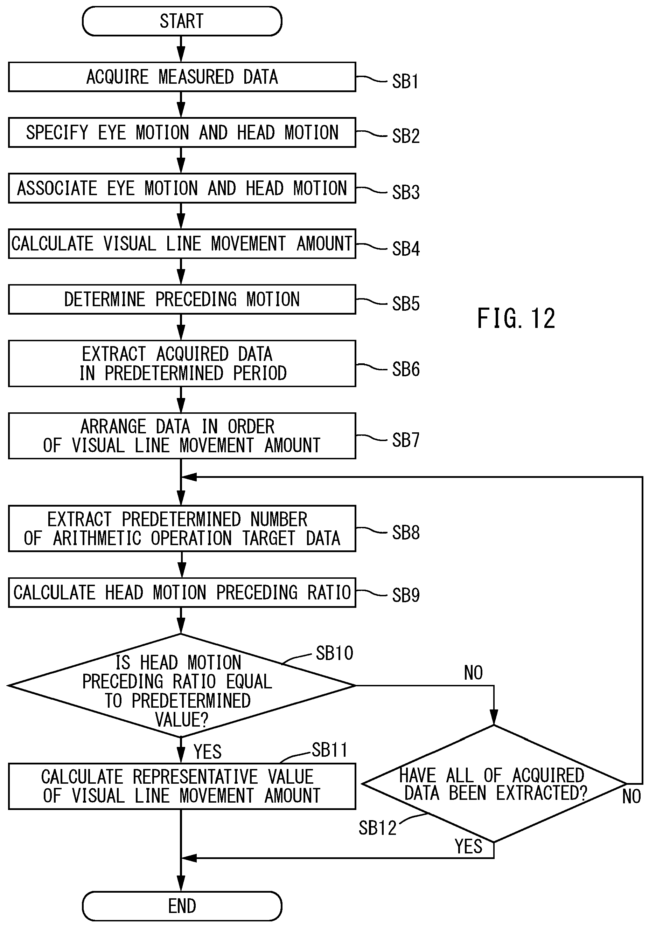

FIG. 11 illustrates a functional block of a visual-line-movement-related-value-acquisition device 120 according to a second embodiment. FIG. 12 illustrates a flowchart of an operation of the visual-line-movement-related-value-acquisition device 120 according to the second embodiment. In this embodiment, unlike the first embodiment, the visual-line-movement-related-value-acquisition device 120 does not generate a relationship between a head motion preceding ratio and a visual line movement amount and outputs, when the head motion preceding ratio is equal to a predetermined value, a corresponding visual line movement amount. In the following description, components similar to the first embodiment are denoted by the same reference signs, the description for the components is omitted, and only different portions from the first embodiment will be described.

### (Structure of Visual-Line-Movement-Related-Value-Acquisition Device)

As illustrated in FIG. 11, the visual-line-movement-related-value-acquisition device 120 includes an arithmetic processor 121, an eye camera 25, a rider gyro sensor 26, a vehicle gyro sensor 27, a wireless communication device 28, and a wireless communication device 29. Note that, although not particularly illustrated, similar to the visual-line-movement-related-value-acquisition device 20 of the first embodiment, the visual-line-movement-related-value-acquisition device 120 of this embodiment also includes a storage.

The arithmetic processor 121 includes a same-subject's-continuous-data-acquisition section 41, a same subject's-visual-line-movement-related-value-acquisition section 42, and a coordinated-motion-related-value-acquisition section 143.

The coordinated-motion-related-value-acquisition section 143 includes a data extraction section 61, a head-motion-preceding-ratio-calculation section 62, and a visual-line-movement-amount-average-calculation section 163.

When the head motion preceding ratio calculated by the head-motion-preceding-ratio-calculation section 62 is equal to a predetermined value, the visual-line-movement-amount-average-calculation section 163 calculates an average value of visual line movement amounts in arithmetic operation target data for which the head motion preceding ratio has been calculated. The calculated average value of the visual line movement amounts is output, as a parameter related to an effective visual field in accordance with an internal state, to an information output section 18 and an ECU 9.

### (Operation of Visual-Line-Movement-Related-Value-Acquisition Device)

Next, an operation of the visual-line-movement-related-value-acquisition device 120 will be described with reference to FIG. 12. Note that, in FIG. 12, Steps SB1 to SB9 are similar to Steps SA1 to SA9 of the flow in the first embodiment, and therefore, the description thereof will be omitted.

In Step SB10, the visual-line-movement-amount-average-calculation section 63 of the coordinated-motion-related-value-acquisition section 143 determines whether the head motion preceding ratio calculated by the head-motion-preceding-ratio-calculation section 62 from a predetermined number of arithmetic operation target data is equal to the predetermined value. Similar to the first embodiment, in the relationship between the head motion preceding ratio and the visual line movement amount, the predetermined value is a value of the head motion preceding ratio with which a difference in the visual line movement amount can be detected in accordance with an internal state of a person. The predetermined value is, for example, 50%.

In Step SB10, when it is determined that the head motion preceding ratio is equal to the predetermined value (YES), the process proceeds to Step SB11 and the visual-line-movement-amount-average-calculation section 63 calculates an average value (a representative value) of the visual line movement amounts in the predetermined number of arithmetic operation target data which have been extracted by the data extraction section 61 and for which the head motion preceding ratio has been calculated by the head-motion-preceding-ratio-calculation section 62. The calculated average value of the visual line movement amounts is output to the information output section 18 and the ECU 9. Thereafter, this flow is terminated (END).

On the other hand, in Step SB10, when it is determined that the head motion preceding ratio is not equal to the predetermined value (NO), the process proceeds to Step SB 12 and the data extraction section 61 determines whether all of the acquired data in a predetermined period have been extracted as arithmetic operation target data.

In Step SB12, when it is determined that all of the acquired data in the predetermined period have been extracted as the arithmetic operation target data (YES), this flow is terminated (END).

On the other hand, in Step SB12, when it is determined that all of the data in the predetermined period have not been extracted (NO), the process returns to Step SB8 and the data extraction section 61 extracts the predetermined number of data from the data in the predetermined period. Note that, similar to the first embodiment, when the predetermined number of data is extracted from the data in the predetermined period, the data extraction section 61 extracts the predetermined number of data from next data for head data of the predetermined number of extracted data.

In this embodiment, the visual line movement amount when the head motion preceding ratio is the predetermined value can be obtained from the relationship between the head motion preceding ratio and the visual line movement amount. Accordingly, when the visual line movement amount differs in accordance with the internal state, the difference can be calculated. Therefore, the index related to the effective visual field can be obtained from the relationship between the head motion preceding ratio and the visual line movement amount.

### [Third Embodiment]

FIG. 14 illustrates a functional block of a visual-line-movement-related-value-acquisition device 220 according to a third embodiment, which represents the present invention. FIG. 15 illustrates a flowchart of an operation of the visual-line-movement-related-value-acquisition device 220 according to the third embodiment. In this embodiment, unlike the first embodiment, the visual-line-movement-related-value-acquisition device 220 does not continuously acquire a same subject's visual line movement-related value, but acquires an effective visual field-related parameter from a database in which a relationship between a subject's visual line movement-related value and a coordinated motion-related value is stored in advance using one or more subject's visual line movement-related values acquired form a subject. In the following description, a similar component to a corresponding component in the first embodiment is denoted by the same reference sign of the corresponding component in the first embodiment, the description for the component is omitted, and only different portions from the first embodiment will be described.

### (Structure of Visual-Line-Movement-Related-Value-Acquisition Device)

As illustrated in FIG. 14, the visual-line-movement-related-value-acquisition device 220 includes an arithmetic processor 221, an eye camera 25, a rider gyro sensor 26, a vehicle gyro sensor 27, a wireless communication device 28, a wireless communication device 29, and a storage 223.

The arithmetic processor 221 includes a subject's-data-acquisition section 241, a subject's-visual-line-movement-related-value-acquisition section 242, a relationship acquisition section 244, and an effective-visual-field-related-parameter-acquisition section 45.

The subject-data-acquisition section 241 acquires, as subject data, each of the measurement results output from the eye camera 25, the rider gyro sensor 26, and the vehicle gyro sensor 27. The subject-data-acquisition section 241 acquires one or more subject data. Except that the one or more subject data are not continuous data, the one or more subject data are similar data to the same subject's continuous data of the first embodiment, and therefore, the detailed description thereof will be omitted.

The subject's-visual-line-movement-related-value-acquisition section 242 acquires one or more subject's visual line movement-related values that are values related to a visual line movement of a subject, based on the one or more subject data acquired by the subject-data-acquisition section 241. The one or more subject's visual line movement-related values are values related to an effective visual field of the subject. Specifically, in this embodiment, the one or more subject's visual line movement-related values are visual line movement amounts of the subject.

The subject's-visual-line-movement-related-value-acquisition section 242 outputs not only the one or more subject's visual line movement-related values but also data including a coordinated motion-related value to the relationship acquisition section 244. The coordinated motion-related value includes information related to head motion and eye motion. Specifically, the subject's-visual-line-movement-related-value-acquisition section 242 also outputs, as the coordinated motion-related value, information on which one of the head motion or the eye motion is preceding motion, information on a time difference (motion time difference) between a start timing of the head motion and a start timing of the eye motion, or the like. Note that, similar to the first embodiment, the coordinated motion-related value may be a head motion preceding ratio.

The relationship acquisition section 244 extracts one data indicating a relationship between the subject's visual line movement-related value and the coordinated motion-related value from the plurality of data stored in the storage 223, based on data output from the subject's-visual-line-movement-related-value-acquisition section 242.

In the storage 223, a plurality of data (for example, graph data, a function expression, table data, or the like) indicating the relationship between the subject's visual line movement-related value and the coordinated motion-related value are stored. Specifically, as the plurality of data, data indicating the relationship between the subject's visual line movement-related value and the coordinated motion-related value in various states of the subject are stored in the storage 223. That is, as the plurality of data, data acquired in advance is stored in the 223. Note that data indicating the relationship between the subject's visual line movement-related value and the coordinated motion-related value in various states of a plurality of subjects may be stored in the storage 223.

Therefore, the relationship acquisition section 244 extracts data indicating the relationship between the subject's visual line movement-related value and the coordinated motion-related value that match the data output from the subject's-visual-line-movement-related-value-acquisition section 242 from the above-described plurality of data stored in the storage 223.

The effective-visual-field-related-parameter-acquisition section 45 acquires a parameter related to the effective visual field of the subject using the data indicating the relationship between the subject's visual line movement-related value and the coordinated motion-related value, the relationship having been extracted by the relationship acquisition section 244. Specifically, the effective-visual-field-related-parameter-acquisition section 45 acquires, as the parameter related to the effective visual field, a visual line movement amount corresponding to a predetermined value of the motion time difference. Note that the predetermined value is a value of the motion time difference with which a difference in the visual line movement amount can be detected in accordance with an internal state of a person in a relationship between the motion time difference and the visual line movement amount.

### (Operation of Visual-Line-Movement-Related-Value-Acquisition Device)

Next, an operation of the visual-line-movement-related-value-acquisition device 220 will be described with reference to FIG. 15.

When a flow illustrated in FIG. 15 starts in Step SC1, the subject-data-acquisition section 241 acquires measured data measured from each of the eye camera 25, the rider gyro sensor 26, and the vehicle gyro sensor 27. Specifically, the subject-data-acquisition section 241 acquires eye motion data related to eye motion of the subject, the eye motion data having been measured by the eye camera 25. Also, the subject-data-acquisition section 241 acquires head motion data related to head motion of the subject, the head motion data having been measured by the rider gyro sensor 26. Also, the subject-data-acquisition section 241 acquires vehicle behavior data related to a behavior of the vehicle 1, the vehicle behavior data having been measured by the vehicle gyro sensor 27.

Next, in Step SC2, the eye-motion-specifying section 51 of the subject's-visual-line-movement-related-value-acquisition section 242 specifies eye motion, based on the eye motion data acquired by the subject-data-acquisition section 241, and also specifies a start timing of the eye motion. Acquisition of eye motion, specification of eye motion, and specification of a start timing of eye motion are performed by a similar method to the method used in the first embodiment, and therefore, the detailed description thereof will be omitted.

In Step SC2, the head-motion-specifying section 52 of the subject's-visual-line-movement-related-value-acquisition section 242 specifies head motion, based on the head motion data acquired by the subject-data-acquisition section 241, and also specifies a start timing of the head motion. Acquisition of head motion, specification of head motion, and specification of a start timing of head motion are performed by a similar method to the method used in the first embodiment, and therefore, the detailed description thereof will be omitted.

In subsequent Step SC3, the motion association section 53 of the subject's-visual-line-movement-related-value-acquisition section 242 associates the eye motion detected by the eye-motion-specifying section 51 and the head motion detected by the head-motion-specifying section 52 with each other at timings at which the motions are specified.

In Step SC4, the visual-line-movement-amount-calculation section 54 of the subject's-visual-line-movement-related-value-acquisition section 242 calculates, as a visual line movement amount, the total of the motion amounts (an integrated value of the rotation angular velocity) in a zone in which each motion is specified in the eye motion and the head motion associated with each other by the motion association section 53.

In Step SC5, the motion-preceding-determination section 55 of the subject's-visual-line-movement-related-value-acquisition section 242 determines preceding motion of the eye motion and the head motion associated with each other by the motion association section 53 and calculates a time difference between the eye motion and the head motion. Determination of preceding motion is performed by a similar method to the method used in the first embodiment, and therefore, the detailed description thereof will be omitted.

A determination result (preceding motion information) of the motion-preceding-determination section 55 is linked to the visual line movement amount calculated by the visual-line-movement-amount-calculation section 54.

**In** Step SC6, the relationship acquisition section 244 extracts data indicating the relationship between the subject's visual line movement-related value and the coordinated motion-related value that match the data output from the subject's-visual-line-movement-related-value-acquisition section 242 from the plurality of data stored in the storage 223.

**In** Step SC7, the effective-visual-field-related-parameter-acquisition section 45 acquires the parameter related to the effective visual field of the rider (the subject) from the relationship between the motion time difference and the visual line movement amount. Specifically, the effective-visual-field-related-parameter-acquisition section 45 acquires, as the parameter related to the effective visual field, the visual line movement amount corresponding to a predetermined value of the motion time difference. Note that the predetermined value is a value of the motion time difference with which a difference in the visual line movement amount can be detected in accordance with the internal state of the person in the relationship between the motion time difference and the visual line movement amount.

Herein, Step SC4 is a subject's visual line movement-related value acquisition step in which a subject's visual line movement-related value related to the visual line movement of the subject is acquired from the subject data.

Step SC5 is a coordinated motion-related value acquisition step in which a coordinated motion-related value related to a coordinated motion of the eye motion and the head motion of the subject for the acquired subject's visual line movement-related value is acquired.

Furthermore, Step SC7 is a subject's visual line movement-related value output step in which the subject's visual line movement-related value related to the visual line movement of the subject when the coordinated motion-related value related to the coordinated motion of the eye motion and the head motion of the subject is a predetermined value is output from a relationship between the acquired subject's visual line movement-related value related to the visual line movement of the subject and the acquired coordinated motion-related value related to the coordinated motion of the eye motion and the head motion of the subject.

According to this embodiment, unlike the first embodiment, the visual line movement amount when the head motion preceding ratio is the predetermined value can be obtained from the relationship between the head motion preceding ratio and the visual line movement amount stored in the storage 223 without continuously measuring the eye motion and the head motion of the same subject. Therefore, the index related to the effective visual field can be easily obtained.

### [Fourth Embodiment]

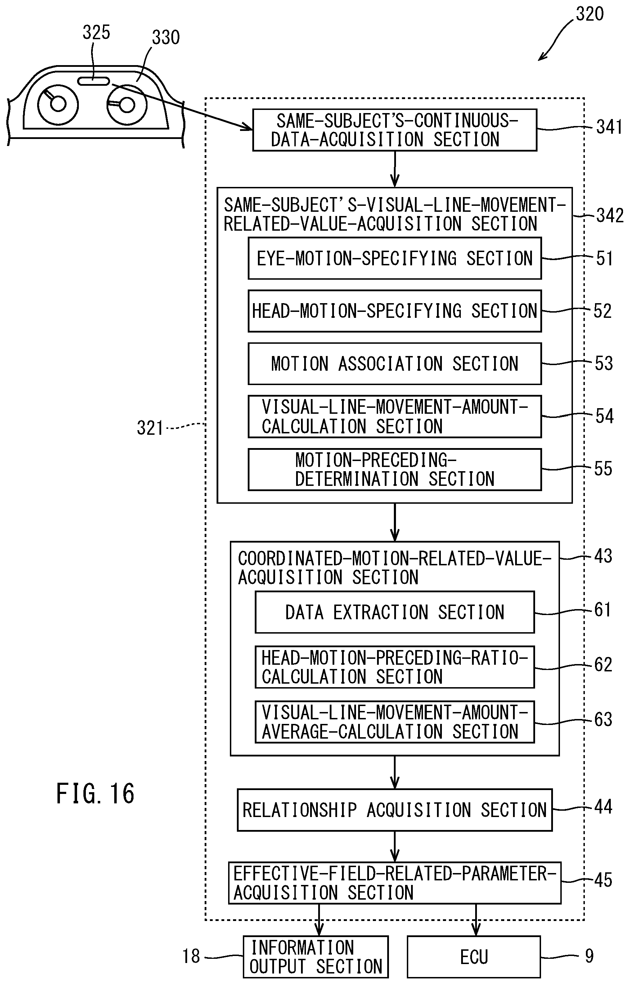

FIG. 16 illustrates a functional block of a visual-line-movement-related-value-acquisition device 320 according to a fourth embodiment. In this embodiment, the visual-line-movement-related-value-acquisition device 320 is different from the structure of the first embodiment in a point that an eye camera 325 that can detect a visual line of a rider is provided in a meter panel of a vehicle. In the following description, a similar component to a corresponding component in the first embodiment is denoted by the same reference sign of the corresponding component in the first embodiment, the description for the component is omitted, and only different portions from the first embodiment will be described.

Similar to the first embodiment, the visual-line-movement-related-value-acquisition device 320 is provided in the vehicle. The visual-line-movement-related-value-acquisition device 320 includes an arithmetic processor 321, a storage (not illustrated), and the eye camera 325.

The eye camera 325 is configured to image eyes and a head of a rider who sits in a rider's seat of a vehicle and thereby be able to measure movement of a visual line and the head of the rider. That is, the eye camera 325 measures movement of a visual line, that is, eye motion, of the rider by imaging movement of the eyes of the rider, and measures head motion by imaging movement of the head of the rider. As described above, in this embodiment, the head motion of the rider is also measured by the eye camera 325 without using a gyro sensor or the like. An image of the rider obtained by the eye camera 325 is image-processed by an arithmetic operation device (not illustrated). The arithmetic operation device obtains measurement results of the eye motion and the head motion of the rider from the image-processed data and outputs the measurement results to the arithmetic processor 321. Note that image processing and data processing of the image obtained by the eye camera 325 may be performed by the arithmetic processor 321.

The eye camera 325 can be used, for example, as a camera that detects movement of the eyes or eyelids of the rider, or the like. The eye camera 325 has a similar structure to that of a typical driver monitoring infrared camera mounted on a vehicle. Therefore, the detailed description of the eye camera 325 will be omitted. Note that the eye camera 325 may be a camera other than the infrared camera as long as the eye camera 325 has a structure capable of detecting the movement of the eyes or the eyelids of the rider.

For example, in a vehicle (not illustrated), the eye camera 325 is provided in a meter panel 330 located in front of a rider's seat of the vehicle. Specifically, the eye camera 325 is provided in an upper portion of the meter panel 330 in order to easily detect the visual line and the head of the rider who sits on the rider's seat of the vehicle. Note that, the eye camera 325 may be provided in a position other than the meter panel 330, as long as the visual line and the head of the rider can be detected in the position.

The arithmetic processor 321 acquires and outputs the visual line movement-related value related to the visual line movement of the rider (the subject), based on the measurement results of the eye camera 325. Specifically, the arithmetic processor 321 includes a same-subject's-continuous-data-acquisition section 341, a same subject's-visual-line-movement-related-value-acquisition section 342 (subject's-visual-line-movement-related-value-acquisition section), a coordinated-motion-related-value-acquisition section 43, a relationship acquisition section 44, and an effective-visual-field-related-parameter-acquisition section (subject's-visual-line-movement-related-value-output section) 45.

The same-subject's-continuous-data-acquisition section 341 acquires each measurement result output from the eye camera 325 as same subject's continuous data. The same subject's continuous data includes eye motion data acquired by continuously measuring the eye motion of a same subject by the eye camera 325 and head motion data acquired by continuously measuring the head motion of the same subject by the eye camera 325.

The same subject's-visual-line-movement-related-value-acquisition section 342 acquires the same subject's visual line movement-related value (the subject's visual line movement-related value) that is a value related to the visual line movement of the subject, based on the same subject's continuous data acquired by the same-subject's-continuous-data-acquisition section 341. Specifically, the same subject's-visual-line-movement-related-value-acquisition section 342 continuously calculates an eye motion amount in the eye motion data of the same subject's continuous data, continuously calculates the head motion amount of the head motion data of the same subject's continuous data corresponding to the eye motion data, and continuously obtains a sum of the eye motion amount and the head motion amount. Also, the same subject's-visual-line-movement-related-value-acquisition section 342 specifies a start timing of the head motion of the subject and a start timing of the eye motion of the subject, and determines which one of the head motion or the eye motion precedes, based on the specified timings.

Similar to the first embodiment, the visual line movement amount of the subject is represented by the total of the eye motion amount and the head motion amount of the subject. That is, also in this embodiment, the sum of the eye motion amount and the head motion amount is the visual line movement amount of the subject. Therefore, the same subject's-visual-line-movement-related-value-acquisition section 342 continuously obtains the visual line movement amount of the subject.

Calculation of the visual line movement amount and preceding determination of motion (eye motion and head motion) in the same subject's-visual-line-movement-related-value-acquisition section 342 are similar to those of the first embodiment. Therefore, the detailed description thereof will be omitted.

The visual line movement amount and preceding motion information acquired by the same subject's-visual-line-movement-related-value-acquisition section 342 are input to the coordinated-motion-related-value-acquisition section 43. Similar to the first embodiment, the coordinated-motion-related-value-acquisition section 43 acquires the coordinated motion-related value related to the coordinated motion of the eye motion and the head motion of the subject, based on data output from the same subject's-visual-line-movement-related-value-acquisition section 342.

Similar to the first embodiment, the relationship acquisition section 44 generates data (for example, a graph, a table, or the like) indicating the relationship between the head motion preceding ratio and the visual line movement amount using the head motion preceding ratio and the average value of the visual line movement amounts output from the coordinated-motion-related-value-acquisition section 43.

Similar to the first embodiment, the effective-visual-field-related-parameter-acquisition section 45 acquires, as the parameters related to the effective visual field, the visual line movement amount X, Y (similar to X, Y in FIG. 8) when the head motion preceding ratio is a predetermined value (for example, 50%), based on data acquired by the relationship acquisition section 44. Note that, also in this embodiment, similar to the first embodiment, the predetermined value may be a value between 40% and 60%. That is, the effective-visual-field-related-parameter-acquisition section 45 may be configured to use any value as the predetermined value as long as the value is a value of the head motion preceding ratio with which a difference in the visual line movement amount can be detected in accordance with the internal state of the person in the relationship between the head motion preceding ratio and the visual line movement amount.

Thus, similar to the first embodiment, the effective visual field of the person can be detected using the parameter acquired by the effective-visual-field-related-parameter-acquisition section 45. Note that the visual line movement amount X, Y acquired by the parameter related to the effective visual field is the same subject's visual line movement-related value (the subject visual line movement-related value) related to the visual line movement of the subject.

Note that the effective-visual-field-related-parameter-acquisition section 45 may be configured to, when there are a plurality of visual line movement amounts that correspond to the predetermined value of the head motion preceding ratio in the data indicating the relationship between the head motion preceding ratio and the visual line movement amount, acquire a value at which the visual line movement amount is the smallest as the parameter related to the effective visual field and also acquire a value at which the visual line movement amount is the largest as the parameter related to the effective visual field. Also, the effective-visual-field-related-parameter-acquisition section 45 may be configured to, when there are a plurality of visual line movement amounts that correspond to the predetermined value of the head motion preceding ratio as described above, acquire an intermediate value of the value at which the visual line movement amount is the smallest and the value at which the visual line movement amount is the largest as the parameter related to the effective visual field and also acquire an average value of the visual line movement amounts as the parameter related to the effective visual field.

Also, the visual line movement amounts X and Y corresponding to the predetermined values of the head motion preceding ratio on an approximate curve of data indicating the relationship between the head motion preceding ratio and the visual line movement amount may be acquired as parameters related to the effective visual field.

Except a point that the eye motion and the head motion of the rider are measured by the eye camera 325 provided in the meter panel 330 of the vehicle instead of the eye camera 25, the rider gyro sensor 26, and the vehicle gyro sensor 27 in the first embodiment, an operation of the visual-line-movement-related-value-acquisition device 320 is similar to the operation of the visual-line-movement-related-value-acquisition device 20 of the first embodiment. Therefore, detailed description of the operation of the visual-line-movement-related-value-acquisition device 320 will be omitted.

As described above, in this embodiment, the eye motion and the head motion of the rider are measured by the eye camera 325 provided in the meter panel 330 of the vehicle and the parameter related to the effective visual field can be acquired based on results of the measurement. Therefore, the parameter related to the effective visual field of the rider can be easily obtained, unlike the first embodiment, without requiring the rider of the vehicle to wear an eye camera and with a compact structure, as compared to the structure of the first embodiment.

Note that the structure of the visual-line-movement-related-value-acquisition devices 120, 220 of the second or third embodiment may be used for the visual-line-movement-related-value-acquisition device 320 of this embodiment.

### [Other Embodiments]

The embodiments of the present teaching have been described above, but the above-described embodiments are merely examples for carrying out the present teaching. Therefore, the present teaching is not limited to the above-described embodiments and the above-described embodiments can be appropriately modified and implemented without departing from the gist of the present teaching.

In each of the above-described embodiments, the same subject's visual line movement-related value is a visual line movement amount of a same subject calculated by the visual-line-movement-amount-calculation section 54. However, the same subject's visual line movement-related value may be some other value as long as the value is a value related to the visual line movement of the same subject, such as, for example, an index related to saccade (saccade size, saccade time, saccade velocity, or the like), an index related to head motion (head motion amount, head motion time, head motion velocity, or the like), or the like.

Note that the saccade size is the same as the eye motion amount and the saccade time means a length of time in a zone in which eye motion is specified by the eye-motion-specifying section 51. The saccade velocity means an average of the eye rotation angular velocity in the zone in which the eye motion is specified by the eye-motion-specifying section 51. The head motion time means a length of time in a zone in which head motion is specified by the head-motion-specifying section 52. The head motion velocity means an average of the head rotation angular velocity in the zone in which the head motion is specified by the head-motion-specifying section 52.

In each of the above-described embodiments, the coordinated motion-related value is the head motion preceding ratio of the same subject calculated by the head-motion-preceding-ratio-calculation section 62. However, the coordinated motion-related value may be some other value as long as the value is a value related to the coordinated motion of the eye motion and the head motion of the same subject, such as, for example, a time difference between a start timing of the head motion and a start timing of the eye motion.

In each of the above-described embodiments, the predetermined number of data which have been extracted by the data extraction section 61 and for which the head motion preceding ratio has been calculated by the head-motion-preceding-ratio-calculation section 62, the average value of visual line movement amounts is a representative value of the visual line movement amounts. However, the representative value of the visual line movement amounts may be any value as long as the value is a value, such as a median in the predetermined number of data or the like, which represents the predetermined number of data.

In each of the above-described embodiments, the time point at which the absolute value of the rotation angular velocity exceeds the threshold α for the first time in the eye motion specified by the eye-motion-specifying section 51 is determined to be a start of the eye motion. The time point at which the absolute value of the rotational angular velocity exceeds the threshold γ for the first time in the head motion specified by the head-motion-specifying section 52 is determined to be a start of the head motion. However, the start of the eye motion may be determined using some other parameter related to the eye motion. Similarly, the start of the head motion may be determined using some other parameter related to the head motion.

In each of the above-described embodiments, the rider gyro sensor 26 is used for measuring head motion of the rider, and the vehicle gyro sensor 27 is used for measuring a behavior of the vehicle 1. However, the rider gyro sensor 26 may be some other sensor, as long as the sensor is capable of measuring the head motion of the rider. The vehicle gyro sensor 27 may be some other sensor, as long as the sensor is capable of measuring the behavior of the vehicle 1. Note that, when an inertial measurement unit (IMU) is used, instead of a gyro sensor, acceleration can be detected.

In the first embodiment, the parameters related to the effective visual field acquired by the visual-line-movement-related-value-acquisition device 20 can be used in determination of the internal state of the subject when the subject exercises or performs an operation of a machine or the like. Also, the acquired parameter can be used in relative evaluation between subjects or the like for the internal state when the subject exercises or performs the operation of the machine. Furthermore, the acquired parameter can be also used in evaluation and management of the internal state of the subject in an operation and a work in a virtual space. Similarly, the parameter related to the effective visual field acquired by the visual-line-movement-related-value-acquisition devices 120, 220, 320 in the second to fourth embodiments can be also used in determination of the internal state of the subject, when the subject exercises or performs an operation of a machine, relative evaluation of the internal state between the subjects, evaluation and management of the internal state of the subject in an operation or work in a virtual space, or the like.

### INDUSTRIAL APPLICABILITY

The present teaching is applicable to a visual-line-movement-related-value-acquisition device that acquires a visual line movement-related value related to a visual line movement of a same subject.

### REFERENCE SIGNS LIST

1 Vehicle (transport apparatus)
2 Vehicle body
7 Seat
8 Power unit
9 ECU
18 Information output section
20, 120, 220, 320 Visual-line-movement-related-value-acquisition device
21, 121, 221, 321 Arithmetic processor
22, 223 Storage
25, 325 Eye camera
26 Rider gyro sensor (head-motion-measurement section)
27 Vehicle gyro sensor (transport-apparatus-motion-measurement section)
28, 29 Wireless communication device
31 Helmet
41, 241, 341 Same-subject's-continuous-data-acquisition section
42, 242, 342 Same subject's-visual-line-movement-related-value-acquisition section (subject's-visual-line-movement-related-value-acquisition section)
43, 143 Coordinated-motion-related-value-acquisition section
44, 244 Relationship acquisition section
45 Effective-visual-field-related-parameter-acquisition section (subject's-visual-line-movement-related-value-output section)
51 Eye-motion-specifying section
52 Head-motion-specifying section
53 Motion association section
54 Visual-line-movement-amount-calculation section
55 Motion-preceding-determination section
61 Data extraction section
62 Head-motion-preceding-ratio-calculation section
63, 163 Visual-line-movement-amount-average-calculation section

## Claims

1. A visual-line-movement-related-value-acquisition device (220) configured to
acquire a value related to a visual line movement of a subject as a subject's visual line movement-related value related from subject's data including a plurality of data related to the subject, and
acquire a coordinated motion-related value related to a coordinated motion of eye motion and head motion of the subject for the acquired subject's visual line movement-related value from the subject's data,
wherein the subject's visual line movement related value is a visual line movement amount of the subject,
wherein the coordinated motion-related value is information on which one of the head motion or the eye motion is preceding motion, or motion time difference that is a time difference between a start timing of the head motion and a start timing of the eye motion in the coordinated motion,
wherein the visual-line-movement-related-value-acquisition device (220)
comprises a database configured to store a relationship between the subject's visual line movement-related value and the coordinated motion-related value in advance, and
configured to extract data indicating the relationship between the subject's visual line movement-related value and the coordinated motion-related value that match the acquired subject's visual line movement-related value,
and
configured to output the subject's visual line movement-related value when the coordinated motion-related value is predetermined value from the extracted relationship between the acquired subject's visual line movement-related value and the acquired coordinated motion-related value, to hereby acquire an index related to an effective visual field of the subject.

2. The visual-line-movement-related-value-acquisition device (220) according to claim 1,
wherein the database is configured to store data indicating the relationship between the subject's visual line movement-related value and the coordinated motion-related value in various states of the subject.

3. The visual-line-movement-related-value-acquisition device (220) according to claim 1 or 2,
wherein the data stored in the database includes graph data, a function expression, and table data that indicate the relationship between the subject's visual line movement-related value and the coordinated motion-related value.

4. A transport apparatus, comprising:
the visual-line-movement-related-value-acquisition device (220) according to any one of claims 1 to 3,
wherein the subject is a rider.

5. The transport apparatus according to claim 4, further comprising:
a transport-apparatus-motion-measurement section (27) configured to measure a motion of the transport apparatus; and
a head-motion-measurement section (26) configured to measure a head motion of the subject,
wherein the visual-line-movement-related-value-acquisition device (220) is configured to exclude the motion of the transport apparatus measured by the transport-apparatus-motion-measurement section (27) from the head motion of the subject measured by the head-motion-measurement section (26) and to calculate the subject's visual line movement-related value and the coordinated motion-related value.

6. The transport apparatus according to claim 4 or 5, further comprising:
an information output section (18) configured to output information corresponding to the subject's visual line movement-related value, the subject's visual line movement-related value having been output from the visual-line-movement-related-value-acquisition device (220).

7. The transport apparatus according to any one of claims 4 to 6, further comprising:
a control device (9) configured to control an operation of the transport apparatus, based on the subject's visual line movement-related value, the subject's visual line movement-related value having been output from the visual-line-movement-related-value-acquisition device (220).

## Patentansprüche

1. Eine Vorrichtung (220) zur Erfassung eines auf eine Sichtlinienbewegung bezogenen Werts, die ausgebildet ist zum
Erfassen eines Werts, der sich auf eine Sichtlinienbewegung einer Person bezieht, als einen sichtlinienbewegungsbezogenen Wert der Person aus Daten der Person, die eine Mehrzahl von Daten in Bezug auf die Person umfassen, und
Erfassen eines auf eine koordinierte Bewegung bezogenen Werts in Bezug auf eine koordinierte Bewegung einer Augenbewegung und einer Kopfbewegung der Person für den erfassten sichtlinienbewegungsbezogenen Wert der Person aus den Daten der Person,
wobei der sichtlinienbewegungsbezogene Wert der Person eine Sichtlinienbewegungsmenge der Person ist,
wobei der auf eine koordinierte Bewegung bezogene Wert eine Information darüber ist, welche der Kopfbewegung oder der Augenbewegung die vorausgehende Bewegung ist, oder eine Bewegungszeitdifferenz ist, die eine Zeitdifferenz zwischen einer Beginnzeitgebung der Kopfbewegung und einer Beginnzeitgebung der Augenbewegung bei der koordinierten Bewegung ist,
wobei die Vorrichtung (220) zur Erfassung eines auf eine Sichtlinienbewegung bezogenen Werts
eine Datenbank aufweist, die dazu konfiguriert ist, eine Beziehung zwischen dem sichtlinienbewegungsbezogenen Wert der Person und dem auf eine koordinierte Bewegung bezogenen Wert im Voraus zu speichern,
und
dazu konfiguriert ist, Daten zu extrahieren, welche die Beziehung zwischen dem sichtlinienbewegungsbezogenen Wert der Person und dem auf eine koordinierte Bewegung bezogenen Wert anzeigen und die mit dem erlangten sichtlinienbewegungsbezogenen Wert der Person übereinstimmen,
und
dazu konfiguriert ist, den sichtlinienbewegungsbezogenen Wert der Person auszugeben, wenn der auf eine koordinierte Bewegung bezogene Wert ein vorbestimmter Wert ist, aus der extrahierten Beziehung zwischen dem erfassten sichtlinienbewegungsbezogenen Wert der Person und dem erfassten auf eine koordinierte Bewegung bezogenen Wert, um hierdurch einen Index in Bezug auf ein tatsächliches Sichtfeld der Person zu erfassen.

2. Die Vorrichtung (220) zur Erfassung eines auf eine Sichtlinienbewegung bezogenen Werts gemäß Anspruch 1,
bei der die Datenbank dazu konfiguriert ist, Daten zu speichern, welche die Beziehung zwischen dem sichtlinienbewegungsbezogenen Wert der Person und dem auf eine koordinierte Bewegung bezogenen Wert in verschiedenen Zuständen der Person zu speichern.

3. Die Vorrichtung (220) zur Erfassung eines auf eine Sichtlinienbewegung bezogenen Werts gemäß Anspruch 1 oder 2,
bei der die in der Datenbank gespeicherten Daten Diagrammdaten, einen Funktionsausdruck und Tabellendaten umfassen, welche die Beziehung zwischen dem sichtlinienbewegungsbezogenen Wert der Person und dem auf eine koordinierte Bewegung bezogenen Wert anzeigen.

4. Ein Transportgerät, das folgende Merkmale aufweist:
die Vorrichtung (220) zur Erfassung eines auf eine Sichtlinienbewegung bezogenen Werts gemäß einem der Ansprüche 1 bis 3,
wobei die Person ein Fahrer ist.

5. Das Transportgerät gemäß Anspruch 4, das ferner folgende Merkmale aufweist:
einen Transportgerätbewegungsmessabschnitt (27), der dazu ausgebildet ist, eine Bewegung des Transportgeräts zu messen; und
einen Kopfbewegungsmessabschnitt (26), der dazu ausgebildet ist, eine Kopfbewegung der Person zu messen,
wobei die Vorrichtung (220) zur Erfassung eines auf eine Sichtlinienbewegung bezogenen Werts dazu ausgebildet ist, die Bewegung des Transportgeräts, die gemessen wird durch den Transportgerätbewegungsmessabschnitt (27), aus der Kopfbewegung der Person auszuschließen, die durch den Kopfbewegungsmessabschnitt (26) gemessen wird, und den sichtlinienbewegungsbezogenen Wert der Person und den auf eine koordinierte Bewegung bezogenen Wert zu berechnen.

6. Das Transportgerät gemäß Anspruch 4 oder 5, das ferner folgendes Merkmal aufweist:
einen Informationsausgabeabschnitt (18), der dazu ausgebildet ist, Informationen auszugeben, die dem sichtlinienbewegungsbezogenen Wert der Person entsprechen, wobei der sichtlinienbewegungsbezogene Wert der Person aus der Vorrichtung (220) zur Erfassung eines auf eine Sichtlinienbewegung bezogenen Werts ausgegeben wurde.

7. Das Transportgerät gemäß einem der Ansprüche 4 bis 6, das ferner folgendes Merkmal aufweist:
eine Steuervorrichtung (9), die dazu ausgebildet ist, einen Betrieb des Transportgeräts zu steuern basierend auf dem sichtlinienbewegungsbezogenen Wert der Person, wobei der sichtlinienbewegungsbezogene Wert der Person aus der Vorrichtung (220) zur Erfassung eines auf eine Sichtlinienbewegung bezogenen Werts ausgegeben wurde.

## Revendications

1. Dispositif d'acquisition de valeur relative au mouvement de ligne visuelle (220) configuré pour
acquérir une valeur relative au mouvement de ligne visuelle d'un sujet en tant que valeur relative au mouvement de ligne visuelle du sujet à partir des données du sujet comportant une pluralité de données relatives au sujet, et
acquérir une valeur relative au mouvement coordonné relative à un mouvement coordonné d'un mouvement des yeux et d'un mouvement de la tête du sujet pour la valeur relative au mouvement de ligne visuelle du sujet acquise à partir des données du sujet,
dans lequel la valeur relative au mouvement de ligne visuelle du sujet est une quantité de mouvement de ligne visuelle du sujet,
dans lequel la valeur relative au mouvement coordonné est une information indiquant lequel du mouvement de la tête ou du mouvement des yeux est le mouvement précédent, ou une différence de temps de mouvement qui est une différence de temps entre un moment de début du mouvement de la tête et un moment de début du mouvement des yeux dans le mouvement coordonné,
dans lequel le dispositif d'acquisition de valeur relative au mouvement de ligne visuelle (220)
comprend une base de données configurée pour stocker à l'avance un rapport entre la valeur relative au mouvement de ligne visuelle du sujet et la valeur relative au mouvement coordonné, et
est configuré pour extraire des données indiquant le rapport entre la valeur relative au mouvement de ligne visuelle du sujet et la valeur relative au mouvement coordonné et correspondant à la valeur relative au mouvement de ligne visuelle du sujet acquise, et
est configuré pour sortir la valeur relative au mouvement de ligne visuelle du sujet lorsque la valeur relative au mouvement coordonné est une valeur prédéterminée du rapport extrait entre la valeur relative au mouvement de ligne visuelle du sujet acquise et la valeur relative au mouvement coordonné acquise, afin d'acquérir ainsi un indice relatif au champ visuel effectif du sujet.

2. Dispositif d'acquisition de valeur relative au mouvement de ligne visuelle (220) selon la revendication 1,
dans lequel la base de données est configurée pour stocker des données indiquant le rapport entre la valeur relative au mouvement de ligne visuelle du sujet et la valeur relative au mouvement coordonné dans divers états du sujet.

3. Dispositif d'acquisition de valeur relative au mouvement de ligne visuelle (220) selon la revendication 1 ou 2,
dans lequel les données stockées dans la base de données comprennent des données graphiques, une expression fonctionnelle et des données tabulaires qui indiquent le rapport entre la valeur relative au mouvement de ligne visuelle du sujet et la valeur relative au mouvement coordonné.

4. Appareil de transport, comprenant :
le dispositif d'acquisition de valeur relative au mouvement de ligne visuelle (220) selon l'une quelconque des revendications 1 à 3,
dans lequel le sujet est un conducteur.

5. Appareil de transport selon la revendication 4, comprenant par ailleurs :
un segment de mesure de mouvement d'appareil de transport (27) configuré pour mesurer un mouvement de l'appareil de transport; et
un segment de mesure du mouvement de tête (26) configuré pour mesurer un mouvement de la tête du sujet,
dans lequel le dispositif d'acquisition de valeur relative au mouvement de ligne visuelle (20, 120, 220, 320) est configuré pour exclure le mouvement de l'appareil de transport mesuré par la segment de mesure de mouvement d'appareil de transport (27) du mouvement de la tête du sujet mesuré par le segment de mesure de mouvement de tête (26) et pour calculer la valeur relative au mouvement de ligne visuelle du sujet et la valeur relative au mouvement coordonné.

6. Appareil de transport selon la revendication 4 ou 5, comprenant par ailleurs :
un segment de sortie d'informations (18) configuré pour sortir les informations correspondant à la valeur relative au mouvement de ligne visuelle du sujet, la valeur relative au mouvement de ligne visuelle du sujet ayant été sortie du dispositif d'acquisition de valeur relative au mouvement de ligne visuelle (220).

7. Appareil de transport selon l'une quelconque des revendications 4 à 6, comprenant par ailleurs :
un dispositif de commande (9) configuré pour commander une opération de l'appareil de transport, sur base de la valeur relative au mouvement de ligne visuelle du sujet, la valeur relative au mouvement de ligne visuelle du sujet ayant été sortie du dispositif d'acquisition de valeur relative au mouvement de ligne visuelle (220).
